# NEW EUROPEAN PATENT SPECIFICATION

(11) **EP 0 701 605 B2**
(45) Date of publication and mention of the opposition decision: **22.02.2017**
(45) Mention of the grant of the patent: 26.03.2008
(21) Application number: 95912559.2
(22) Date of filing: 23.02.1995
(51) Int. Cl.: C12N 15/57, C12N 9/52, C12N 9/54, C12N 9/56, C11D 3/386

(54) **IMPROVED ENZYMES AND DETERGENTS CONTAINING THEM**
VERBESSERTE ENZYME UND DIESE ENTHALTENE DETERGENTIEN
ENZYMES AMELIOREES ET DETERGENTS LES CONTENANT

(30) Priority: 24.02.1994 US 201120; 17.01.1995 US 373818
(43) Date of publication of application: 20.03.1996
(60) Divisional application: 07023832.4
(73) Proprietor: BASF SE, 67056 Ludwigshafen am Rhein (DE)
(72) Inventor: CHRISTIANSON, Teresa M., Petaluma, CA 94954 (US); GODDETTE, Dean W., Chelmsford, MA 01824 (US); KOTTWITZ, Beatrix, D-40593 Düsseldorf (DE); MAURER, Karl-Heinz, D-40699 Erkrath (DE); PAECH, Christian G., Daly City, CA 94014 (US); POCHANDKE, Winfried, D-40789 Monheim (DE); POETHKOW, Joerg, D-40229 Düsseldorf (DE); SCHMIDT, Irmgard, D-42697 Solingen (DE); TANG, Maria R., Fairfield, CA 94533 (US); UPADEK, Horst, D-40883 Ratingen (DE); WEISS, Albrecht, D-40764 Langenfeld (DE); WILSON, Charles Ronald, Santa Rosa, CA 95401 (US)
(74) Representative: BASF IP Association
(86) International application number: PCT/US1995/001937
(87) International publication number: WO 1995/023221

(56) References cited:
- EP-A- 0 405 901
- WO-A-94/02618
- WO-A-95/30011
- US-A- 5 340 735
- US-A- 5 352 604
- Proceedings of the National Academy of Sciences USA, Volume 84, issued August 1987, WELLS et al., "Recruitment of Substrate Specificity Properties from one Enzyme into a Related one by Protein Engineering", pages 5167-5171, see entire document.
- Journal of Molecular Biology, Volume 228, issued 1992, GODDETTE et al., "The Crystal Structure of the Bacillus Lentus Alkaline Protease, Subtilisin BL, at 1.4 Angstrom Resolution", pages 580-595, see entire document.

## Description

### BACKGROUND OF THE INVENTION

### 1. Field of the Invention

This invention relates to novel mutant proteolytic enzymes with improved properties relative to the wild type enzyme in cleaning and detergent formulations, to nucleotide sequences encoding the improved proteases, and to host organisms containing the nucleotide sequences encoding the novel proteases. This invention includes within its scope new and improved detergent and cleansing compositions containing an effective cleansing amount of said enzymes.

### 2. Description of the Related Art

Subtilisins are a family of bacterial extracellular proteases with molecular masses of 20,000 to 45,000 daltons produced by a soil bacillus e.g. *Bacillus amyloliquefaciens*. Proteases are enzymes which catalyze the hydrolysis of peptide linkages in protein and peptide substrates and of ester bonds in some terminal esters. Subtilisins belong to the group of serine proteases which initiate the nucleophilic attack on the peptide (ester) bond by a serine residue at the active site. Subtilisins are physically and chemically well characterized enzymes. The three-dimensional structure of several subtilisins has been elucidated in detail by X-ray diffraction studies (Betzel, C., Pal, G.P., and Saenger, W. (1988) Eur. J. Biochem. 178, 155-171; Bott, R., Ultsch, M., Kossiakoff, A., Graycar, T., Katz, B., and Power, S. (1988) J. Biol. Chem. 263, 7895-7906; Goddette, D.W., Paech, C., Yang, S.S., Mielenz, J.R., Bystroff, C., Wilke, M., and Fletterick, R.J. (1992) J. Mol. Biol. 228, 580-595; Heinz. D.W., Priestle, J.P., Rahuel, J., Wilson, KS., and Grütter. M.G. (1991) J. Mol. Biol. 217, 353-371; Kraut, J. (1977) Annu. Rev. Biochem. 46, 331-358; Neidhart, D.J. and Petsko, G.A. (1988) Protein Eng. 2, 271-276; Teplyakov, A.V., Kuranova, I.P., Harutyunyan, E.H., Vainshtein, B.K., Frömmel, C., Höhne, W.-E., and Wilson, K.S. (1990) J. Mol. Biol. 214, 261-279). In spite of this wealth of information the structure/function differences between these closely related subtilisins have not been explained.

Subtilisins are widely used in commercial products (for example, in laundry and dish washing detergents, contact lens cleaners) and for research purposes (catalysts in synthetic organic chemistry). One member of the subtilisin family, a highly alkaline protease for use in detergent formulations has been described in patent application WO 91/02792. This *Bacillus lentus* alkaline protease (BLAP) can be obtained in commercial quantities from *Bacillus licheniformis* ATCC 53926 strain transformed by an expression plasmid harboring the wild type BLAP gene under the control of the *B. licheniformis* ATCC 53926 alkaline protease gene promoter. The crystal structure of BLAP has been deduced (Goddette, D.W., et al. (1992) J. Mol. Biol. 228, 580-595; WO 92/21760), and the coordinates have been deposited with the Brookhaven Protein Data Bank. Unless other wise noted the numbering of the amino acid positions is according to the sequence in BLAP (269 amino acids), which differs from that of subtilisin BPN' (275 amino acids). When aligned for optimal sequence homology the following pattern emerges. In BLAP positions 1 to 35, 36 to 54, 55 to 160, and 161 to 269 correspond to positions 1 to 35, 37 to 55, 57 to 162, and 167 to 275, respectively, in subtilisin BPN'.

In order to describe protease variants according to the invention, the following nomenclature is used: [Original Amino Acid; Position from N-terminus of the mature enzyme; Substituted Amino Acid]. For example, the substitution of valine with isoleucine at position 4 in BLAP is designated as V41. The list of abbreviations for amino acids is shown in Table 1.

**TABLE 1**

| **Amino Acid Nomenclature** | | | | |
|---|---|---|---|---|
| A | = | Ala | = | Alanine |
| C | = | Cys | = | Cysteine |
| D | = | Asp | = | Aspartic acid |
| E | = | Glu | = | Glutamic acid |
| F | = | Phe | = | Phenylalanine |
| G | = | Gly | = | Glycine |
| H | = | His | = | Histidine |
| I | = | Ile | = | Isoleucine |
| K | = | Lys | = | Lysine |
| L | = | Leu | = | Leucine |
| M | = | Met | = | Methionine |
| N | = | Asn | = | Asparagine |
| P | = | Pro | = | Proline |
| Q | = | Gln | = | Glutamine |
| R | = | Arg | = | Arginine |
| S | = | Ser | = | Serine |
| T | = | Thr | = | Threonine |
| V | = | Val | = | Valine |
| W | = | Trp | = | Tryptophan |
| Y | = | Tyr | = | Tyrosine |

Multiple mutations within the same protein molecule are indicated as the sum of individual mutations, i.e. S3T + V41 + A188P + V193M + V1991.

Protection against thermal and chemical inactivation and improvement of washing and cleaning performance are primary objectives in research and development of proteases for technical as well as for commercial applications. A large number of enzymes including subtilisins have been generated by random and site-specific mutagenesis and they provide some guidelines for a rational approach to the improvement of thermal and chemical stability (Estell, D.A., Graycar, T.P., and Wells, J.A. (1985) J. Biol. Chem. 260, 6518-6521; Matsumura, M., Becktel, W.J., Levitt, M., and Matthews, B.W. (1989) Proc. Natl. Acad. Sci. USA 86, 6562-6566; Pantoliano, M.W., Whitlow, M., Wood, J.F., Rollence, M.L., Finzel, B.C., Gilliland, G.L., Poulos, T.L., and Bryan, P.N. (1988) Biochemistry 27, 8311-8317; Russell, A.J. and Fersht, A.R. (1987) Nature 328, 496-500; Siezen, R.J., De Vos, W.M., Leunissen, J.A.M., and Dijkstra, B.W. (1991) Protein Eng. 4, 719-737; van Ee, J.H. (1991) Chimicaoggi (7/8). 31-35; Wells, J.A. and Estell, D.A. (1988) Trends Biochem. Sci. 13, 291-297). The modulation of enzymic activity, in particular rate enhancement or optimization for a subset of substrates, is a far more complex problem. EP 0260105 teaches the construction of subtilisin BPN' mutants with altered transesterification rate/hydrolysis rate ratios and nucleophile specificities by changing specific amino acid residues within 15 A of the catalytic triad. Russell, A.J., and Fersht, A.R. (1987) J. Mol. Biol. 193: 803-813, teach the isolation of a subtilisin BPN' mutant (D099S) that had a change in the surface charge 14 to 15Å from the active site. This substitution causes an effect on the pH dependence of the subtilisin's catalytic reaction. Neither of these publications teach how to predict amino acid alterations that will improve the wash performance of the protease. EP 0130756, EP 0247647, and US 4,760,025 teach a saturation mutation method where one or multiple mutations are introduced into the subtilisin BPN' at amino acid residues (BPN' numbering) Asp32, Asn155, Tyr104, Met222, Gly166, His64, Ser221, Gly169, Glu156, Ser33, Phe189, Tyr217, and/or Ala152. Using this approach mutant proteases exhibiting improved oxidative stability, altered substrate specificity, and/or altered pH activity are obtained. These publications also teach that mutations within the active site region of the protease are the most likely to influence activity. However, neither EP0130756, EP 0247647, nor US 4,760,025 teach a method for predicting amino acid alterations that will improve the wash performance of the protease.

Most of the information on the catalytic activity of subtilisins has been collected by examining the hydrolysis of small, well defined peptide substrates. Yet, little is known about interactions with large protein substrates. This is especially true for the wash performance of proteases where the substrate is attached to a textile surface and catalysis takes place in presence of interfering compounds such as bleach, tensides, and builders.

EP 0328229 teaches the isolation and characterization of PB92 subtilisin mutants with improved properties for laundry detergent applications based upon wash test results. It teaches that biochemical properties are not reliable parameters for predicting enzyme performance in the wash. Methods for selection of mutations involve the substitution of amino acids by other amino acids in the same category (polar, nonpolar, aromatic, charged, aliphatic, and neutral), the substitution of polar amino acids asparagine and glutamine by charged amino acids, and increasing the anionic character of the protease at sites not involved with the active site. No method for identifying which specific amino acids should be altered is taught. Patent application WO 91/00345 (Novo-Nordisk) teaches a method to improve the wash performance of a subtilisin by the modification of the isoelectric point of subtilisin Carlsberg and subtilisin 309 to match the pH of the washing solution, where the enzyme is supposed to be used. However, since the highly alkaline subtilisins have pl values close to the pH of detergents under European washing conditions, this approach offers no obvious advantage. WO 91/00345 also teaches that changes in amino acids more than 15 Å from the catalytic triad can result in changes in the kinetic properties of the enzyme. A total of 116 different amino acids out of a total of 269 amino acids present in subtilisin 309 are suggested as possible sites for substitution, addition or deletion to modify the net electric charge of the enzyme.

Patent application WO 92/11357 (Novo-Nordisk) teaches increased pH-stability and improved washability of subtilisins by reduction of pH-dependent charges of the molecule. This means the introduction of mutations to approach substantial constancy of charge over a pH range. Preferably, an almost zero net charge change in the pH range from 7 to 11. European patent application No. 0 57 049 A1 discloses certain mutant proteolytic enzymes. These enzymes are said to have at least 70% homology with the amino acid sequence of PB92 serine protease and differ by at least one amino acid corresponding to 99, 102, 116, 126, 127, 128, 130, 160, 203, 211, and 212 in the PB92 sering protease. The mutant protease is prepared by growing a microorganism host strain transformed with an expression vector comprising a DNA sequence and encoding a mutant protease to produce the designed mutant protease.

The proteases hitherto proposed for use in detergents and washing aids were selected for high proteolytic activity. On account of their low specificity, therefore, fiber damage or fiber destruction by the protease can occur in particular in the event of repeated washing of fabrics of proteinogenic fibers, for example sheet-form textiles of silk or wool. On the other hand, any reduction in protease activity results in a loss of cleaning power noticeable to the user of the detergent. Accordingly, one problem addressed by the present invention was to develop protease-containing detergents which would show reduced activity on proteinogenic fibers, more particularly wool, for substantially the same washing activity. Another problem addressed by the present invention was to develop soil-specific proteases which are proteases that remove specific stains on fabrics such as eggs stains and blood stains and detergent formulations containing such soil-specific proteases.

The wild-type protease from which the mutant proteases according to the invention are derived is a *Bacillus lentus* alkaline protease (BLAP) obtained from DSM 5483 having 269 amino acid residues, a molecular mass of 26,823 daltons and a calculated isoelectric point of 9.7 based on standard pK values. The BLAP gene is obtained by isolating the chromosomal DNA from the *B. lentus* strain DSM 5483, constructing DNA probes having homology to putative DNA sequences encoding regions of the *B. lentus* protease, preparing genomic libraries from the isolated chromosomal DNA and screening the libraries for the gene of interest by hybridization to the probes.

Mutants of *B. lentus* DSM 5483 protease with improved thermal and surfactant stability have been described in patent application Serial No. 07/706,691 filed May 29, 1991. In general the mutations described in this invention are introduced into wild-type BLAP with the following amino acid replacements: S3T, V41, A188P, V193M, V199I and L211D (numbering according to the BLAP sequence).

Research directed towards the binding of the protease to the high molecular protein substrate on the textile revealed the importance of the charged amino acids situated in the proximity of the substrate binding site. It could be shown that the removal of positively charged amino acid residues or the introduction of negatively charged amino acid residues in the region of the substrate binding pocket of the protease leads towards better wash performance compared to the wild type.

The present invention also relates to detergent compositions for washing fabrics composed of proteinogenic fibers such as wool without causing damage to such fibers. The detergents are comprised of at least one surfactant and a proteolytically active amount of a protease having a keratinase/caseinase activity ratio of less than about 0.80.

Another aspect of the present invention relates to detegent compositions which are particularly effective in removing stains such as blood and egg stains and especially combinations of such stains from fabrics. The detergents are comprised of a combination of proteases which are Bacillus lentus DSM 5483 variants and at least one surfactant.

### BRIEF DESCRIPTION OF THE FIGURES

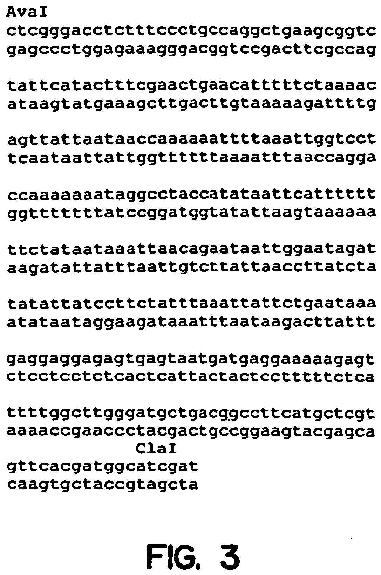

**Figure 3** shows the DNA sequence for the *Ava*I/*Cla*I fragment from the N-terminal region of the ATCC 53926 alkaline protease gene discussed in Example 2. The fragment includes the putative promoter, ribosomal binding site, initiation codon, and most of the pre sequence. The 292 base pair fragment is flanked by *Ava*I and *Cla*I restriction sites at its 5' and 3' ends, respectively.

### DEPOSIT OF MICROORGANISMS

*Bacillus licheniformis* E312 with plasmid pBC56M131 is deposited as **ATCC 68614.** *Escherichia coli* WK6 with plasmid pMC13C is deposited as **ATCC 68615.** *E. coli* GM33 with plasmid pCB13C is deposited as **ATCC 68616.** *E. coli* WK6 with plasmid pMa5-8 is deposited as **ATCC 68617**. *E. coli* WK6 with pMc5-8 is deposited as **ATCC 68618.**

### Detailed Description of Aspects of the Invention

The invention is defined by the claims only. Certain aspects useful for understanding the invention and the technical field thereof are described hereinafter.

One aspect of the present invention relates to a mutant protease with improved wash performance. Improved wash performance is obtained by introducing amino acid alterations within a region of the substrate binding pocket of the enzyme which results in an increased negative charge. According to the present invention, this can be achieved by increasing the number of negatively charged amino acids residues or decreasing the number of positively charged amino acid residue in the region of the substrate binding pocket of the protease within 7A of a bound substrate molecule such as AAPF. In particular, amino acid alterations at positions 99, 154 and 211 within *Bacillus lentus* Alkaline Protease (BLAP) variants M130 and M131 were shown to enhance the wash performance of the enzyme.

A second aspect of the present invention relates to mutant proteolytic enzymes which have an improved wash performance relative to the wild type protease as determined by laboratory tests. The mutations described in this invention are introduced into BLAP variants M130 or M131 which have been previously described in patent application Serial No. 07/706,691 filed May 29, 1991. Both M130 and M131 have been shown to have improved stability as compared to the wild type protease. Mutant M130 contains four amino acid alterations: S3T; A188P/ V193M and V1991. Mutant M131 contains five amino acid alterations: S37; V41; A188P; V193M and V1991. The system used to designate preferred proteases first list the amino acid residue in the mature form of BLAP at the numbered position followed by the replacement amino acid using the accepted one letter amino acid codes. The amino acid sequences for proteases M130 and M131 are given in SEQ ID NO: 2 and SEQ ID NO: 1, respectively, contained in patent application Serial No. 07/706,691 filed May 29, 1991. Both M130 and M131 served as the basis for additional amino acid alterations to achieve proteases with improved wash performance. The mutant proteases according to the invention are characterized by at least one amino acid alteration which results in a reduced positive charge or an increased negative charge in the region of the substrate binding pocket, wherein said amino acid alteration is L211D according to the counting of SEQ ID No. 22. In another embodiment, the mutant proteases are those derived by the replacement of at least one amino acid residue of the mutant proteases M130 or M131 wherein said amino acid residue is selected from the group consisting of arginine at position 99 or serine at position 154 in addition to leucine at position 211. Table 2 provides a description of the BLAP mutant proteases which include F11, F43, F44, F45, F46, F47, F49, F54 and F55. For example F11 shown in Table 2 is derived from M130 and contains an arginine at position 99 replaced by a serine (R99S) along with the other mutations present in M130 which include: a serine at position 3 replaced by a threonine (S3T); an alanine at position 188 replaced by a proline (A188P); a valine at position 193 replaced by a methionine (V193M) and a valine at position 199 replaced by a isoleucine (V1991). Of the remaining mutant proteases, F43 through F49 are derived from M131. Mutants F54 and F55 are derived from mutant F49. The amino acid sequences of the preferred proteolytic enzymes F11, F43, F44, F45, F46, F47, F49, F54 and F55 are given in SEQ ID NO:10 to SEQ ID NO:18, respectively, of this application.

Another aspect of this invention relates to the genes which encode the mutant proteases. The genetic construction of all mutant proteases in this invention are described in detail in Example 2. In all cases the mutations introducing the amino acid alterations are constructed using known procedures. The DNA sequences encoding the mature forms of the referred proteases F11, F43, F44, F45, F46, F47, F49, F54 and F55. Each hybrid gene encoding one of the mutant proteases listed above is comprised in the direction of transcription, a promoter, a ribosomal binding site, and initiation codon and the major portion of the pre region of the *B. licheniformis* ATCC 53926 alkaline protease gene operably linked to a portion of the pre region and all of the pro and characterized by at least one amino acid alteration which results in a reduced positive charge or an increased negative charge in the region of the substrate binding pocket, wherein said amino acid alteration is L211D according to the counting of SEQ ID No. 22. In another embodiment, the mutant proteases are mature regions of a variant of the BLAP gene followed by the transcription terminator sequence for the alkaline protease gene from ATCC 53926. The hybrid gene may be integrated into the chromosome of the host or is carried on a plasmid which replicates within the *Bacillus* strain of choice. In particular, plasmid pUB110 or a derivative of pUB110 is the plasmid of choice for protease production in strains of *Bacillus subtilis* and plasmid pBC16 or a derivative of pBC16 is the choice for protease production in strains of *B. licheniformis.* The mutant proteases are produced by growing the Bacillus strains transformed by plasmids containing the hybrid genes in a suitable medium.

Another aspect of the present invention relates to detergent compositions which contain at least one surfactant and a proteolytically active quantity of a protease having a keratinase/caseinase activity ratio (hereinafter referred to as the KC ratio) of less than about 0.80, preferably less than about 0.70 and more preferably in the range from about 0.2 to about 0.65.

The detergent preferably has a proteolytic activity in the range from about 100 PU/g to around 7500 PU/g.

The present invention also relates to the use of proteases with a KC ratio of less than about 0.80, preferably less than about 0.70 and more preferably in the range from about 0.2 to about 0.65 for washing textiles of proteinogenic fibers or textiles consisting at least partly of proteinogenic fibers.

The KC ratio includes the activity of the protease with respect to two different substrates, casein and keratin.

The proteolytic activity of a protease solution which, in the case of casein as substrate, produces an absorption of 0.500 OD under the described measuring conditions, is defined as 10 PU (protease units) per ml. The cleaning result observed by the user of a protease-containing detergent correlates with this activity expressed in PU. The observed damage to a fabric of proteinogenic fibers correlates with the KC ratio for a constant cleaning result.

As expected in the light of the problem stated above, an only minimal difference in the activities towards casein and keratin was observed in the case of commercial proteases, the KC ratio being relatively close to 1. It was found that proteases with KC ratios of less than 0.80 have to be used to achieve a no longer significantly noticeable degree of damage to proteinogenic fabric.

It has surprisingly been found that proteases having the properties mentioned above can synergistically influence the effect of certain other detergent ingredients and that, conversely, the proteolytic activity of the protease can be synergistically enhanced by certain other detergent ingredients. These effects occur in particular in the case where the surfactant component of the compositions according to the invention contain nonionic surfactants, soil-release copolyesters (more particularly those containing terephthalic acid units), water-insoluble organic builders, water-soluble inorganic and organic builders (more particularly based on oxidized carbohydrates) and synthetic anionic surfactants of the sulfate and sulfonate type, but not very strongly - if at all - in the case of alkylbenzenesulfonates. Detergents according to the invention preferably contain ingredients of which the properties are enhanced or which enhance the properties of the protease.

The proteases suitable for use in accordance with the invention include genetically engineered variants of Bacillus lentus DSM 5483. These variants can be prepared according to the method set forth in US patent application serial number 08/201,120 filed on 02/24/94 and in US patent number 5,340,735. Preferred variants of Bacillus lentus DSM 5483 (BLAP) are listed in Table 2 in Example 2.

In reference to Table 2, the system used to identify the variants is the same as that used in US patent number 5,340,735 and explained therein. For example, mutant F49is identified as S3T + V41 + A188P + V 193M + V199I + L211D. Such a protease is a mutant Bacillus lentus DSM 5483 variant wherein the serine residue at position 3 is replaced by threonine, the valine residue at position 4 is replaced by isoleucine, the alanine residue at position 188 is replaced by proline, the valine residue at position 193 is replaced by methionine, the valine residue at position 199 is replaced by isoleucine, and the leucine residue at position 211 is replaced by aspartic acid. The amino acid sequence of the wild-type BLAP protease is set forth as sequence ID number 52 in US patent number 5,340,735.

It has been found that some Bacillus lentus DSM 5483 variants showed preferential activity toward blood stains while others showed preferential activity toward egg stains. Mutants that are particularly effective in removing blood and egg stains from fabrics are those mutants made by making the following replacements in the wild-type Bacillus lentus DSM 5483 protein: R99G, R99A, R99S, S154D, S154E, L221 D. Mutants having replacement amino acid residues at positions 99 and 154 are particularly effective in removing blood stains. Mutants having replacement amino acid residues at position 211 are particularly effective in removing egg stains. It has been found that a combination of mutant enzymes having replacement amino acid residues at positions 99 and 154 and those having replacement amino acid residues at position 211 are particularly effective in removing a combination of blood and egg stains

Therefore another aspect of the present invention relates to detergent compositions which are particularly effective in removing combinations of stains such as blood and egg stains from fabrics. The detergents are comprised of a combination of proteases which are Bacillus lentus DSM 5483 variants and a nonionic surfactant, an anionic surfactant, a soap, or a combination of such surfactants. Protease combinations include any two or more of the Bacillus lentus DSM 5483 variants listed in the Table above. A particularly preferred combination is F46 + F49 for removing stains comprised of blood and egg stains.

In one preferred embodiment, a detergent according to the invention contains nonionic surfactants selected from fatty alkyl polyglycosides, fatty alkyl polyalkoxylates, more particularly ethoxylates and/or propoxylates, fatty acid polyhydroxyamides and/or ethoxylation and/or propoxylation products of fatty alkylamines, vicinal diols, fatty acid alkyl esters and/or fatty acid amides and mixtures thereof, more particularly in a quantity of 2% by weight to 25% by weight.

In another preferred embodiment, a detergent according to the invention contains synthetic anionic surfactant of the sulfate and/or sulfonate type, more particularly fatty alkyl sulfate, fatty alkyl ether sulfate, sulfofatty acid esters and/or sulfofatty acid disalts, more particularly in a quantity of 2% by weight to 25% by weight. The anionic surfactant is preferably selected from the alkyl or alkenyl sulfates and/or the alkyl or alkenyl ether sulfates in which the alkyl or alkenyl group contains 8 to 22 carbon atoms and, more particularly, 12 to 18 carbon atoms.

In another preferred embodiment, a detergent according to the invention contains water-soluble and/or water-insoluble builders, more particularly selected from alkali metal alumosilicate, crystalline alkali metal silicate with a modulus of > 1, monomeric polycarboxylate, polymeric polycarboxylate and mixtures thereof, more particularly in quantities of 2.5% by weight to 60% by weight.

Another, albeit less preferred, embodiment of a detergent according to the invention intended for washing wool or silk fabrics may contain peroxygen-based bleaching agents, more particularly hydrogen peroxide, alkali metal perborate tetrahydrate, alkali metal perborate monohydrate and/or alkali metal percarbonate, more particularly in quantities of 5% by weight to 70% by weight, and optionally bleach activators, more particularly in quantities of 2% by weight to 10% by weight.

Finally, another embodiment of a detergent according to the invention contains soil-release agents based on copolyesters of dicarboxylic acids and glycols which may be present in particular in quantities of 0.01 % by weight to 5% by weight. These soil-release agents, which are particularly effective by virtue of their chemical similarity to polyester fibers, but are also capable of developing the required effect in fabrics of other materials are copolyesters containing dicarboxylic acid units, alkylene glycol units and polyalkylene glycol units. Soil-release copolyesters of the type mentioned and their use in detergents have been known for some time.

The detergent compositions according to the invention contain at least one surfactant which includes soaps. The surfactants can be nonionic or anionic surfactants or a combination of nonionic and anionic surfactants or a combination of nonionic and anionic surfactants and one or more soaps.

Suitable nonionic surfactants include the alkoxylates, more particularly ethoxylates and/or propoxylates, of saturated or mono- to polyunsaturated linear or branched alcohols containing 10 to 22 carbon atoms and preferably 12 to 18 carbon atoms. The degree of alkoxylation of the alcohols is generally between 1 and 20 and preferably between 3 and 10. They may be obtained in known manner by reaction of the corresponding alcohols with the corresponding alkylene oxides. Derivatives of the fatty alcohols are particularly suitable, although their branched-chain isomers, more particularly so-called oxoalcohols, may also be used for the production of useful alkoxylates. Accordingly, the alkoxylates, more particularly ethoxylates, of primary alcohols containing linear groups, more particularly dodecyl, tetradecyl, hexadecyl or octadecyl groups, and mixtures thereof may be used. In addition, corresponding alkoxylation products of alkylamines, vicinal diols and carboxylic acid amides corresponding to the above-mentioned alcohols in regard to the alkyl moiety are also suitable. Alkyl polyglycosides suitable for incorporation in the detergents according to the invention are compounds corresponding to the general formula (G)ₙ-OR¹, where R¹ is an alkyl or alkenyl radical containing 8 to 22 carbon atoms, G is a glycose unit and n is a number of 1 to 10. The glycoside component (G)ₙ is an oligomer or polymer of naturally occurring aldose or ketose monomers, including in particular glucose, mannose, fructose, galactose, talose, gulose, altrose, allose, idose, ribose, arabinose, xylose and lyxose. The oligomers consisting of such glucoside-bonded monomers are characterized not only by the type of sugars present in them, but also by their number, the so-called degree of oligomerization. As an analytically determined quantity, the degree of oligomerization n is generally a broken number which may assume a value of 1 to 10 and, in the case of the glycosides preferably used, a value below 1.5 and, more particularly, between 1.2 and 1.4. Glucose is the preferred monomer unit by virtue of its ready availability. The alkyl or alkenyl substituent R¹ of the glycosides also preferably emanates from readily accessible derivatives of renewable raw materials, more particularly from fatty alcohols, although branched-chain isomers thereof, more particularly so-called oxoalcohols, may also be used for the production of useful glycosides. Accordingly, primary alcohols with linear octyl, decyl, dodecyl, tetradecyl, hexadecyl or octadecyl groups and mixtures thereof are particularly suitable. Particularly preferred alkyl glycosides contain a cocofatty alkyl group, i.e. mixtures in which - essentially - R¹ is dodecyl and R¹ is tetradecyl.

The nonionic surfactant is preferably present in the detergents according to the invention in quantities of 1 % by weight to 30% by weight and more preferably in quantities of 1% by weight to 25% by weight.

In addition to or instead of the nonionic surfactant, the detergents according to the invention may contain other surfactants, preferably synthetic anionic surfactants of the sulfate or sulfonate type, in quantities of preferably not more than 20% by weight and, more preferably, in quantities of 0.1 % by weight to 18% by weight, based on the detergent as a whole. Synthetic anionic surfactants particularly suitable for use in the detergents according to the invention are the alkyl and/or alkenyl sulfates containing 8 to 22 carbon atoms which contain an alkali metal, ammonium or alkyl- or hydroxyalkyl-substituted ammonium ion as counterion. Derivatives of fatty alcohols containing 12 to 18 carbon atoms and branched-chain analogs thereof, so-called oxoalcohols, are preferred. The alkyl and alkenyl sulfates may be prepared in known manner by reaction of the corresponding alcohol component with a typical sulfating agent, more particularly sulfur trioxide or chlorosulfonic acid, and subsequent neutralization with alkali metal, ammonium or alkyl- or hydroxyalkyl-substituted ammonium bases. Alkyl and/or alkenyl sulfates such as these are preferably present in the detergents according to the invention in quantities of 0.1 % by weight to 20% by weight and more preferably in quantities of 0.5% by weight to 18% by weight.

Suitable surfactants of the sulfate type also include the sulfated alkoxylation products of the alcohols mentioned, so-called ether sulfates. These ether sulfates preferably contain 2 to 30 and, more particularly, 4 to 10 ethylene glycol groups per molecule. Suitable anionic surfactants of the sulfonate type include the α-sulfoesters obtainable by reaction of fatty acid esters with sulfur trioxide and subsequent neutralization, more particularly the sulfonation products derived from C₈₋₂₂ and preferably C₁₂₋₁₈ fatty acids and C₁₋₆ and preferably C₁₋₄ linear alcohols, and the sulfofatty acids emanating from these sulfonation products through formal saponification.

Other optional surface-active ingredients include soaps; suitable soaps being saturated fatty acid soaps, such as the salts of lauric acid, myristic acid, palmitic acid or stearic acid, and soaps derived from natural fatty acid mixtures, for example coconut oil, palm kernel oil or tallow fatty acids. Soap mixtures of which 50% by weight to 100% by weight consist of saturated C₁₂₋₁₈ fatty acid soaps and up to 50% by weight of oleic acid soap are particularly preferred. Soap is preferably present in quantities of 0.1% by weight to 5% by weight. However, larger quantities of soap of up to 20% by weight may be present in particular in liquid detergents.

A detergent according to the invention preferably contains 20% by weight to 55% by weight of water-soluble and/or water-insoluble, organic and/or inorganic builders. Water-soluble organic builders include, in particular, those from the class of polycarboxylic acids, more particularly citric acid and sugar acids, and the class of polymeric (poly) carboxylic acids, more particularly the polycarboxylates obtainable by oxidation of polysaccharides, polymeric acrylic acids, methacrylic acids, maleic acids and copolymers thereof which may even contain small amounts of polymerizable substances with no carboxylic acid functionality in copolymerized form. The relative molecular weight of the homopolymers of unsaturated carboxylic acids is generally in the range from 5,000 to 200,000 while the relative molecular weight of the copolymers is in the range from 2,000 to 200,000 and preferably in the range from 50,000 to 120,000, based on free acid. A particularly preferred acrylic acid/maleic acid copolymer has a relative molecular weight of 50,000 to 100,000. Suitable, but less preferred compounds of this class are copolymers of acrylic acid or methacrylic acid with vinyl ethers, such as vinylmethyl ethers, vinyl esters, ethylene, propylene and styrene, in which the acid makes up at least 50% by weight. Other suitable water-soluble organic builders are terpolymers containing two carboxylic acids and/or salts thereof as monomers and vinyl alcohol and/or a vinyl alcohol derivative or a carbohydrate as the third monomer. The first acidic monomer or its salt is derived from a monoethylenically unsaturated C₃₋₈ carboxylic acid and preferably from a C₃₋₄ monocarboxylic acid, more particularly from (meth)acrylic acid. The second acidic monomer or its salt may be a derivative of a C₄₋₈ dicarboxylic acid, preferably a C₄₋₈ dicarboxylic acid, maleic acid being particularly preferred. In this case, the third monomer unit is formed by vinyl alcohol and/or preferably by an esterified vinyl alcohol. Vinyl alcohol derivatives in the form of an ester of short-chain carboxylic acids, preferably C₁₋₄ carboxylic acids, with vinyl alcohol are particularly preferred. Preferred terpolymers contain 60% by weight to 95% by weight and, more particularly, 70% by weight to 90% by weight of (meth)acrylic acid or (meth)acrylate, more preferably acrylic acid or acrylate, and maleic acid or maleate and 5% by weight to 40% by weight and preferably 10% by weight to 30% by weight of vinyl alcohol and/or vinyl acetate. Terpolymers in which the ratio by weight of (meth)acrylic acid or (meth)acrylate to maleic acid or maleate is between 1:1 and 4:1, preferably between 2:1 and 3:1 and more preferably between 2:1 and 2.5:1 are most particularly preferred (both the quantities and the ratios by weight being based on the acids). The second acidic monomer or its salt may even be a derivative of an allyl sulfonic acid substituted in the 2-position by an alkyl radical, preferably by a C₁₋₄ alkyl radical, or by an aromatic radical preferably derived from benzene or benzene derivatives. Preferred terpolymers contain 40% by weight to 60% by weight and more particularly 45% by weight to 55% by weight of (meth)acrylic acid or (meth)acrylate, more preferably acrylic acid or acrylate, 10% by weight to 30% by weight and preferably 15% by weight to 25% by weight of methallyl sulfonic acid or methallyl sulfonate and, as the third monomer, 15% by weight to 40% by weight and preferably 20% by weight to 40% by weight of a carbohydrate. This carbohydrate may be, for example, a mono-, di-, oligo- or polysaccharide, mono-, di- or oligosaccharides being preferred, sucrose being particularly preferred. Weak points responsible for the ready biodegradability of the polymer are presumably incorporated therein through the use of the third monomer. These terpolymers generally have a relative molecular weight in the range from 1,000 to 200,000, preferably in the range from 200 to 50,000 and more preferably in the range from 3,000 to 10,000. They may be used in the form of aqueous solutions, preferably 30 to 50% by weight aqueous solutions, particularly for the production of liquid detergents. All the polycarboxylic acids mentioned are generally used in the form of their water-soluble salts, more particularly their alkali metal salts.

Organic builders of the type in question are preferably present in quantities of up to 40% by weight, more preferably in quantities of up to 25% by weight and most preferably in quantities of 1 % by weight to 5% by weight. Quantities near the upper limit mentioned are preferably used in paste-form or liquid, more particularly water-containing, detergents according to the invention.

Crystalline or amorphous alkali metal alumosilicates in particular are used as the water-insoluble, water-dispersible inorganic builders in quantities of up to 50% by weight and preferably in quantities of not more than 40% by weight and, in liquid detergents, in quantities of 1 % by weight to 5% by weight. Among these alumosilicates, crystalline sodium alumosilicates in detergent quality, more particularly zeolite A, P and optionally X, are preferred. Quantities near the upper limit mentioned are preferably used in solid particulate detergents. Suitable alumosilicates contain no particles larger than 30 µm in size, at least 80% by weight consisting of particles below 10 µm in size. Their calcium binding capacity is generally in the range from 100 to 200 mg CaO per gram.

Suitable substitutes or partial substitutes for the alumosilicate mentioned are crystalline alkali metal silicates which may be present either on their own or in admixture with amorphous silicates. The alkali metal silicates suitable for use as builders in the detergents according to the invention preferably have a molar ratio of alkali metal oxide to SiO₂ of less than 0.95 and, more particularly, in the range from 1:1.1 to 1:12 and may be present in amorphous or crystalline form. Preferred alkali metal silicates are the sodium silicates, more particularly amorphous sodium silicates, with a molar ratio of Na₂O to SiO₂ of 1:2 to 1:2.8. Amorphous alkali metal silicates such as these are commercially available, for example, as PORTIL®. Those with a molar ratio of Na₂O to SiO₂ of 1:1.9 to 1:2.8 are preferably used in solid form and not in the form of a solution in the production of detergents according to the invention. Preferred crystalline silicates, which may be present either on their own or in admixture with amorphous silicates, are crystalline layer silicates with the general formula Na₂SiₓO₂ₓ₊₁·yH₂O, in which x - the so-called modulus - is a number of 1.9 to 4 and y is a number of 0 to 20, preferred values for x being 2, 3 or 4. Preferred crystalline layer silicates are those in which x in the general formula assumes the value 2 or 3. Both β- and δ-sodium disilicates (Na₂Si₂O₅·yH₂O) are particularly preferred. δ-Sodium silicates with a modulus of 1.9 to 3.2 are preferred. Substantially water-free crystalline alkali metal silicates corresponding to the above general formula, in which x is a number of 1.9 to 2.1 may also be used in detergents according to the invention. Another preferred embodiment of detergents according to the invention are crystalline sodium layer silicate having a modulus of 2 to 3. Crystalline sodium silicates with a modulus of 1.9 to 3.5 are used in another preferred embodiment of detergents according to the invention. The alkali metal silicate content of the detergents according to the invention is preferably 1% by weight to 50% by weight and more preferably 5% by weight to 35% by weight, based on anhydrous active substance. If an alkali metal alumosilicate, more particularly zeolite, is also present as an additional builder, the alkali metal silicate content is preferably 1% by weight to 15% by weight and more preferably 2% by weight to 8% by weight, based on anhydrous active substance. In that case, the ratio by weight of alumosilicate to silicate, based on anhydrous active substances, is preferably 4:1 to 10:1. In detergents containing both amorphous and crystalline alkali metal silicates, the ratio by weight of amorphous alkali metal silicate to crystalline alkali metal silicate is preferably 1:2 to 2:1 and more preferably 1:1 to 2:1.

In addition to the inorganic builder mentioned, other water-soluble or water-insoluble inorganic substances may be used in the detergents according to the invention. Alkali metal carbonates, alkali metal hydrogen carbonates and alkali metal sulfates and mixtures thereof are suitable in this regard. The additional inorganic material may be present in quantities of up to 70% by weight, but is preferably absent altogether.

To adjust an optionally acidic or mildly alkaline pH value of, in particular, about 8.0 to 9.5 in a 1% by weight aqueous solution, the detergents according to the invention may contain solid inorganic and/or organic acids or acidic salts, for example alkali metal hydrogen sulfates, succinic acid, adipic acid or glutaric acid and mixtures thereof. Acidic substances such as these are preferably present in the detergents according to the invention in quantities of not more than 5% by weight and, more particularly, in quantities of 0.1 % by weight to 3% by weight.

The detergents according to the invention may additionally contain other typical detergent ingredients. These optional ingredients include, in particular, complexing agents for heavy metals, for example aminopolycarboxylic acids, aminohydroxypolycarboxylic acids, polyphosphonic acids and/or aminopolyphosphonic acids, redeposition inhibitors, for example cellulose ethers, dye transfer inhibitors, for example polyvinyl pyrrolidones, foam inhibitors, for example organopolysiloxanes or paraffins, solvents and optical brighteners, for example stilbene disulfonic acid derivatives. The detergents according to the invention preferably contain up to 1 % by weight and, more particularly, 0.01% by weight to 0.5% by weight of optical brighteners, more particularly compounds from the class of substituted 4,4'-bis-(2,4,6-triamino-s-triazinyl)-stilbene-2,2'-disulfonic acids, up to 5% by weight and, more particularly, 0.1% by weight to 2% by weight of complexing agents for heavy metals, more particularly aminoalkylene phosphonic acids and salts thereof, up to 3% by weight and, more particularly, 0.5% by weight to 2% by weight of deposition inhibitors and up to 2% by weight and, more particularly, 0.1% by weight to 1% by weight of foam inhibitors, the percentages by weight mentioned being based on the detergent as a whole.

Besides water, solvents which are used in particular in liquid detergents according to the invention are preferably water-miscible solvents, including-lower alcohols, for example ethanol, propanol, isopropanol and isomeric butanols, glycerol, lower glycols, for example ethylene and propylene glycol, and the ethers derived from the classes of compounds mentioned.

The typical enzyme stabilizers optionally present, more particularly in liquid detergents according to the invention, include aminoalcohols, for example mono-, di-, triethanolamine and propanolamine and mixtures thereof, the lower carboxylic acids, boric acid or alkali metal borates, and boric acid/carboxylic acid combinations.

Suitable foam inhibitors include long-chain soaps, more particularly behenic soap, fatty acid amides, paraffins, waxes, microcrystalline waxes, organopolysiloxanes and mixtures thereof which, in addition, may contain microfine, optionally silanized or otherwise hydrophobicized silica. For use in particulate detergents according to the invention, foam inhibitors such as these are preferably deposited on granular water-soluble supports.

In addition, the detergent according to the invention may contain redeposition inhibitors. The function of redeposition inhibitors is to keep the soil detached from the fibers suspended in the liquor and thus to prevent discoloration of the fibers. Suitable redeposition inhibitors are water-soluble, generally organic colloids, for example the water-soluble salts of polymeric carboxylic acids, glue, gelatine, salts of ether carboxylic acids or ether sulfonic acids or starch or cellulose or salts of acidic sulfuric acid esters of cellulose or starch. Water-soluble polyamides containing acidic groups are also suitable for this purpose. Soluble starch preparations and other starch products than those mentioned above, for example partly hydrolyzed starch, may also be used. Sodium carboxymethyl cellulose, methyl cellulose, methyl hydroxyethyl cellulose and mixtures thereof are preferably used.

The bleaching agents which may be used as further ingredients of detergents according to the invention are the per compounds generally used in detergents, such as perborate which may be present as tetrahydrate or monohydrate, percarbonate, perpyrophosphate and persilicate which are generally present in the form of alkali metal salts, more particularly sodium salts. Bleaching agents such as these are preferably present in detergents according to the invention in quantities of up to 25% by weight, more preferably in quantities of up to 15% by weight and most preferably in quantities of 5% by weight to 15% by weight, based on the detergent as a whole.

The bleach activators present as an optional component include the N-acyl or O-acyl compounds typically used, for example polyacylated alkylenediamines, more particularly tetraacetyl ethylenediamine, acylated glycolurils, more particularly tetraacetyl glycoluril, N-acylated hydantoins, hydrazides, triazoles, urazoles, diketopiperazines, sulfurylamides and cyanurates, also carboxylic anhydrides, more particularly phthalic anhydride, carboxylic acid esters, more particularly sodium isononanoyl phenol sulfonate, and acylated sugar derivatives, more particularly pentaacetyl glucose. To avoid interaction with the per compounds during storage, the bleach activators may have been coated with shell-forming substances or granulated in known manner, tetraacetyl ethylenediamine granulated with carboxymethyl cellulose with average particle sizes of 0.01 mm to 0.8 mm, and/or granulated 1,5-diacetyl-2,4-dioxohexahydro-1,3,5-triazine, being particularly preferred. The bleach activators are preferably present in detergents according to the invention in quantities of up to 8% by weight and more particularly in quantities of 2% by weight to 6% by weight, based on the detergent as a whole.

Enzymes optionally present in addition to protease include, in particular, enzymes from the class of lipases, cutinases, amylases, cellulases, pullulanases, oxidases and peroxidases and mixtures thereof. Enzymes obtained from fungi or bacterial strains are preferably used.

The amylases suitable for use in detergents according to the invention include the enzymes obtainable from bacteria or fungi which have a pH optimum preferably in the alkaline range up to about pH 10. Useful commercial products are, for example, TERMAMYL® and MAXAMYL®. Amylase is preferably used in the detergent according to the invention in such quantities that the final detergent contains 0.01 KNU/g to 2 KNU/g ("Kilo Novo Units" per gram according to the standard method of the Novo Company, 1 KNU being the quantity of enzyme which degrades 5.26 kg of starch at pH 5.6/37°C as determined by the method described by Methods in Enzymology, Vol. 1, 1955, page 149), preferably 0.015 KNU/g to 1.8 KNU/g and more preferably 0.03 KNU/g to 1.6 KNU/g.

The cellulase suitable for use in accordance with the invention also belongs to the enzymes obtainable from bacteria or fungi which have a pH optimum preferably in the almost neutral to mildly alkaline pH range from 6 to 9.5. They are preferably used in the detergent according to the invention in such quantities that the final detergent has a cellulolytic activity of 0.05 IU/g to 1.5 IU/g ("International Units" per gram, based on the enzymatic hydrolysis of Na carboxymethyl cellulose at pH 9.0/40°C, preferably 0.07 IU/g to 1.4 IU/g and more preferably 0.1 IU/g to 1.3 IU/g. Suitable commercial products are, for example, CELLUZYME®, a product of Novo Nordisk, or KAC®, a product of Kao.

The enzymes may be adsorbed in known manner onto supports, encapsulated in shell-forming substances and/or granulated in conjunction with support substances to make then easier to handle and to protect them against premature inactivation if they are to be incorporated in particulate detergents. The enzymes optionally present in addition to protease are preferably present in detergents according to the invention in quantities of up to 2% by weight and more preferably in quantities of 0.01 % by weight to 1.5% by weight, based on the detergent as a whole.

In one preferred embodiment, a detergent according to the invention is particulate and contains 20% by weight to 55% by weight of inorganic builder, up to 15% by weight and more particularly from 2% by weight to 12% by weight of water-soluble organic builder, 2.5% by weight to 20% by weight of synthetic anionic surfactant, 1% by weight to 20% by weight of nonionic surfactant, up to 25% by weight and, more particularly, from 1 % by weight to 15% by weight of bleaching agent, up to 8% by weight and, more particularly, from 0.5% by weight to 6% by weight of bleach activator and up to 20% by weight and, more particularly, from 0.1 % by weight to 15% by weight of inorganic salts, more particularly alkali metal carbonate and/or sulfate, and up to 2% by weight and, more particularly, from 0.4% by weight to 1.2% by weight of particulate enzymes besides protease, more particularly lipase, cutinase, amylase, cellulase, pullulanase, oxidase and/or peroxidase.

In another preferred embodiment, a powder-form detergent according to the invention intended in particular for use as a light-duty detergent contains 20% by weight to 55% by weight of inorganic builders, up to 15% by weight and, more particularly, from 2% by weight to 12% by weight of water-soluble organic builders, from 4% by weight to 24% by weight of nonionic surfactant, up to 15% by weight and, more particularly, from 1% by weight to 10% by weight of synthetic anionic surfactant, up to 65% by weight and, more particularly, from 1 % by weight to 30% by weight of inorganic salts, more particularly alkali metal carbonate and/or sulfate, and neither bleaching agents nor bleach activators.

Another preferred embodiment of the detergent according to the invention is a liquid detergent containing 5% by weight to 35% by weight of water-soluble organic builders, up to 15% by weight and, more particularly, from 0.1% by weight to 5% by weight of water-insoluble inorganic builders, up to 15% by weight and, more particularly, from 0.5% by weight to 10% by weight of synthetic anionic surfactant, from 1% by weight to 25% by weight of nonionic surfactant, up to 15% by weight and, more particularly, from 4% by weight to 12% by weight of soap and up to 30% by weight and, more particularly, from 1% by weight to 25% by weight of water and/or water-miscible solvent and also up to 10% by weight and, more particularly, from 0.01 % by weight to 7.5% by weight of an enzyme stabilizing system. The protease in each of the above preferred embodiments is variant F49 having the amino acid substitutions S3T + V41 + A188P + V193M + V1991 + L211D.

Particulate detergents according to the invention may be produced very easily by mixing the individual particles in a standard mixer, more particularly a drum mixer, roller mixer, ribbon mixer or free-fall mixer, other optional powder-form components and, if desired, even liquid or liquefied components, including in particular nonionic surfactants, but also dyes and fragrances, optionally being incorporated by spraying. The components capable of withstanding high temperatures are preferably converted into a powder-form product in known manner by spray drying of an aqueous slurry and the powder obtained is mixed with protease and, optionally, other enzymatic substances and other heat-sensitive components, including in particular bleaching agents. The incorporation of individual constituents by mixing in granules or an extrudate containing them is also possible and is particularly preferred for the production of detergents according to the invention with high apparent densities of, preferably, 650 g/l to 900 g/l. Flowable or liquid detergents according to the invention may be produced simply by mixing the ingredients or compounds thereof which may be present in liquid form or in the form of a solution in water or a given solvent.

### Example 1

### Identification of Sites in BLAP for Mutagenesis

A variety of natural subtilisins and other serine proteases were tested for their washing efficacy. The results showed that washability relates to the number and distribution of charged amino acid residues in the substrate binding region. An improvement in the wash performance was seen with an increased number of negatively charged amino acid residues or with a decrease of the number of positively charged amino acid residues in the substrate binding region. Accordingly, the BLAP mutants claimed herein have a reduced positive net charge in the substrate binding region and they show improved washability.

Improved wash performance was obtained by introducing amino acid alterations within the substrate binding pocket which resulted in a variation in charge. Alterations were made for amino acids residing within the substrate binding pocket of either BLAP variant M130 or BLAP variant M131. The substrate binding pocket of these enzymes is defined as the region within 7 A of a bound substrate molecule, AAPF. AAPF bound to the enzyme was modeled on crystallographic data of subtilisin-inhibitor complexes found in the literature and available from the Brookhaven Protein Data Bank. The structure of BLAP variants M130 and M131 within the substrate binding pocket is essentially identical to wild type BLAP based upon Xray crystallography. The structure of wild type BLAP to a 1.4A resolution has been published in patent application Serial No. 07/706,691 filed May 29, 1991 and the corresponding atomic coordinates deposited with the Brookhaven Protein Data Bank. In particular, amino acid alterations at positions 99, 154 and 211 within BLAP variants M130 and M131 were shown to enhance the wash performance of the enzyme. The parental amino acids occupying these sites, Arginine 99, Serine 154, and Leucine 211 are depicted in Figure 1. All three amino acids are located within the specified 7A radius of AAPF.

### Example 2

### Construction of Mutant Genes of the BLAP Gene

### A. Construction of Mutants M130 and M131

Genes which express the mutant *B. lentus* DSM 5483 proteases according to the invention are made by altering one or more codons of the wild-type *B. lentus* DSM 5483 alkaline protease gene. Protease M130 was derived from BLAP by introducing mutations S3T, A188P, V193M and V199I. Protease M131 was derived from BLAP by introducing the mutations S3T, V4I, A188P, V193M, and V199I. Genes encoding proteases M130 and M131 were constructed using the pMac procedure (Stanssens, P., Opsomer, C., McKeown, Y.M., Kramer, W., Zabeau, M., and Fritz, H.-J. (1989) Nucleic Acids Res. 17, 4441-4445). Proteases M130 and M131 have been previously described in patent application Serial No. 07/706,691 filed May 29, 1991. Proteases M130 and M131 exhibit improved thermal and surfactant stability over the wild type BLAP and served as the basis for developing proteases with improved wash performance. The genetic techniques used to modify the BLAP gene to produce the M130 and M131 proteases have also been described in patent application WO 91/02792.

Proteases M130 and M131 were derived from the *B. lentus* DSM 5483 alkaline protease (BLAP) by site-specific mutagenesis of DNA encoding the mature form of wild type BLAP. The DNA fragment encoding the mature form of wild type BLAP was prepared using plasmid pCB13C. Plasmid pCB13C contains a hybrid fusion between the *B. licheniformis* ATCC 53926 protease gene and the *B. lentus* DSM 5483 BLAP gene Specifically, this hybrid fusion contains DNA encoding the promoter, ribosomal binding site, and 21 residues of the pre sequence from the ATCC 53926 protease gene fused to a DNA sequence encoding the last five residues of the BLAP pre sequence and all of the pro and mature residues of BLAP. This fusion is referred to as the *Cla*I fusion because this restriction site is located at the juncture between the ATCC 53926 and DSM 5483 DNA's. A new *Cla*I restriction site had to be introduced into the ATCC 53926 alkaline protease gene near to the junction of the pre and pro sequences. The C/al site was introduced into the ATCC 53926 alkaline protease gene by using the polymerase chain reaction (PCR) to amplify a DNA fragment containing sequence information from the N-terminal part of the ATCC 53926 alkaline protease gene. The amplified fragment included the ATCC 53926 alkaline protease promoter, ribosomal binding site, initiation codon, and most of the pre sequence. The DNA sequence of this fragment is shown in Figure 3. This 292 bp DNA fragment was flanked by Aval and C/al restriction sites at its 5' and 3' ends, respectively. The BLAP gene already contained a naturally occurring C/al site at the corresponding position. Analysis of the DNA sequence across the fusion of the ATCC 53926 and BLAP genes confirmed the expected DNA and amino acid sequences.

To perform mutagenesis the BLAP gene is subcloned into the mutagenesis vector pMa5-8. This is accomplished by synthesizing a DNA fragment containing the *Cla*I fusion gene and the ATCC 53926 transcription terminator as a *Sal*I cassette using the PCR. The PCR was carried out using conditions as described by the manufacturer (Perkin Elmer Cetus, Norwalk, CT). In the PCR, two synthetic oligonucleotides bearing *Sal*I sites are used as primers and *Escherichia coli* vector pCB13C DNA as a template. After cutting the PCR product with Sa*l*I, this fragment is cloned into the mutagenic plasmid pMc5-8 which has previously been cut with *Sal*I and dephosphorylated with bacterial alkaline phosphatase. Plasmids pMc5-8, and pMa5-8 were obtained from H.-J. Fritz and are described by Stanssens, P., et al. (1989) Nucleic Acids Res. 17, 4441-4454. *Sa*/I sites are chosen to allow the PCR fragment to be cloned into pMc5-8 in both orientations. The ligation mix is transformed into E. *coli* WK6. Chloramphenicol resistant (Cm^{R}) transformants are screened for the presence of an insert and a correct plasmid construct pMc13C is identified. Once the gene is cloned into the pMc5-8 vector and desirable sites for mutation are identified, the mutation(s) is introduced using synthetic DNA oligonucleotides according to a modification of a published protocol (Stanssens, P., et. al. (1989) Nucleic Acids Res. 17, 4441-4454). The oligonucleotide containing the mutation(s) to be introduced is annealed to a gapped duplex (gd) structure which carries the BLAP gene on a segment of single stranded (ss) DNA. The gapped duplex can be formed by annealing linear ss DNA from pMc13C with denatured and restricted pMa5-8 DNA. Plasmid pMa5-8 contains an active ampicillin resistance gene but has an inactivating point mutation in the chloramphenicol resistance gene, whereas plasmid pMc13C contains, in addition to an intact BLAP gene, an active chloramphenicol resistance gene, but has an inactivating point mutation in the ampicillin resistance gene. The annealed product is the gd DNA which is a double stranded heteroduplex with a ss DNA gap spanning the entire cloned BLAP gene. The mutant oligonucleotide is able to anneal to homologous ss BLAP DNA within the gap and the remaining gap is filled in by DNA polymerase I (Klenow fragment) and ligated using T4 DNA ligase (New England Biolabs Inc., Beverly, MA [NEB]). The mutagenic efficiency of such a system can be improved by the use of Exonuclease III (*Exo* III, NEB). *Exo* III is an exodeoxyribonuclease that digests double stranded DNA from the 3' end. As a free 3' end is required, closed circular ss DNA or ds DNA is unaffected by this enzyme. A subsequent treatment of the product of the fill-in reaction with Exo III removes any species with only partially filled gaps. This significantly improves the mutagenic efficiency and is the preferred mutagenesis method. The product of the fill-in reaction is then transformed into a repair deficient *E. coli* strain (WK6mutS) and ampicillin resistant transformants (Ap^{R}) are selected. Replication of the transformed heteroduplex plasmid results in two different progenies. One progeny contains the wild type BLAP gene and the intact chloramphenicol resistance gene, but an inactive ampicillin resistance gene. The other progeny contains a BLAP gene carrying the mutation of interest and is resistant to ampicillin but not to chloramphenicol.

Selection of Ap^{R}, Cm^{S} mutant transformants with ampicillin is not sufficient to stop some background growth of the Ap^{S}, Cm^{R} progeny carrying the wild type BLAP gene. Therefore, it is necessary to perform a second transformation into *E. coli* using plasmid DNA prepared from the Ap^{R} transformants of the WK6mutS strain. This second transformation uses a low plasmid concentration with a large number of recipient cells of a suppressor deficient strain of *E. coli* such as WK6. This approach decreases the likelihood of a recipient cell receiving plasmid DNA from both progeny. Ap^{R} transformants are selected and plasmid DNA from several transformants is isolated and screened for the presence of the mutation. The pMa mutant derivative of the first mutagenesis round can be used for a second round of mutagenesis by preparing ss DNA of that species and annealing it to *Xba*I/*Hind*III restricted and denatured DNA of pMc5-8. Plasmid pMc5-8 is identical to pMa5-8 except that it contains an active chloramphenicol resistance gene and an inactive ampicillin resistance gene. The general procedure is the same as that described above. The construction of the genes encoding proteases M130 and M131 required two rounds of mutagenesis. In the first round of mutagenesis an oligonucleotide was designed to introduce mutations A188P, V193M, and V1991. In a second round of mutagenesis an oligonucleotide was designed to introduce mutation S3T in the case of M130 and mutations S3T and V41 in the case of M131. The presence of all of these mutations was verified by DNA sequencing.

### B. Construction of Genes Encoding New Proteases with Improved Wash Performance.

Mutations R99G, R99A, R99S, S154D, S154E and L211D were introduced into the gene encoding M131 protease using PCR mutagenesis by overlap extension. Mutation R99S was introduced into the gene encoding protease M130 using PCR mutagenesis by overlap extension. Construct pCB76M131 was used to construct mutants F43, F44, F45, F46, F47, and F49 (Table 2). Construct pCB76M130 was used to construct mutant F11 and construct pCB76F49 (a newly constructed mutant) was used to construct mutants F54 and F55.

**TABLE 2**

| **Description of BLAP Mutants** | |
|---|---|
| BLAP | wild type enzyme |
| M130 | S3T + A188P + V193M + V1991 |
| M131 | S3T + V41 + A188P + V193M + V1991 |
| F11 | S3T + R99S + A188P + V193M + V1991 |
| F43 | S3T + V41 + R99G + A188P + V193M + V1991 |
| F44 | S3T + V41 + R99A + A188P + V193M + V1991 |
| F45 | S3T + V41 + R99S + A188P + V193M + V1991 |
| F46 | S3T + V41 + S154E + A188P + V193M + V1991 |
| F47 | S3T + V41 + S154D + A188P + V193M + V1991 |
| F49 | S3T + V4I + A188P + V193M + V1991 + L211D |
| F54 | S3T + V41 + R99G + A188P + V193M + V199I + L211D |
| F55 | S3T + V41 + S154E + A188P + V193M + V1991 + L211D |

Materials required to perform this procedure include: A DNA Thermal Cycler (Perkin Elmer Cetus); GeneAmp kit (Perkin Elmer Cetus); AmpliWax (Perkin Elmer Cetus); and 0.5 ml sterile polypropylene tubes (Perkin Elmer Cetus); Microcon-100 concentrators (Amicon, Beverly, MA); TE buffer (10 mM tris-(hydroxymethyl)aminomethane [Tris], 1 mM disodium ethylenediamine tetraacetic acid (EDTA), adjusted to pH 8 with 2 N HCl); Minigel electrophoresis apparatus (Hoefer Scientific, San Francisco, CA); 1% (w/v) SeaKem agarose gel (FMC, Rockland, ME) in TBE buffer (0.089 M Tris, 0.089 M boric acid, 2 mM EDTA); 0.5 µg·ml⁻¹ ethidium bromide in water; restriction enzymes NheI (NEB), XbaI (NEB); and SstI (BRL, Gaithersburg, MD).

PCR's were carried out using the GeneAmp kit and AmpliWax as specified by the manufacturer. They were subjected to 1 cycle of denaturation (3 minutes, 95°C), annealing (2 minutes, 50°C) and extension (2 minutes, 72°C) and 30 cycles of denaturation (1 minute, 94°C), annealing (1 minutes, 50°C) and extension (1 minute, 72°C) using a DNA Thermal Cycler. Each cycle was extended for 10 sec at 72°C.

Site-directed mutagenesis by overlap extension is described by (Ho, S.N., Hunt, H.D., Horton, R.M., Pullen, J.K, and Pease, L.R. (1989) Gene 77, 51-59). For BLAP mutants F43, F44, F45, F46, F47 and F49 10 ng of pCB76M131 plasmid DNA was used as template for the initial round of mutagenesis. For BLAP mutant F11 10 ng of pCB76M130 plasmid DNA was used as template, and for BLAP mutants F54 and F55 10 ng of pCB76F49 DNA was used as template. The pUB110 forms of these plasmids were chosen because they provide higher yields of protease in the *B. subtilis* DB104 host than the pBC16 forms of the plasmids. PCR fragments were checked by agarose gel electrophoresis and cleaned using a Microcon-100 concentrator (Amicon) after each round of PCR. After the second round of PCR, the fragments were digested with either NheI/XbaI or NheI/SstI (depending on the location of the intended mutation) and cloned back into pCB76M131 DNA in the case of mutants F43, F44, F45, F46, F47 and F49, pCB76M130 DNA in the case of mutant F11 and pCB76F49 in the case of mutants F54 and F55 using *B. subtilis* DB104 competent cells as previously described. Plasmid DNA isolation was accomplished using ion exchange minicolumns (QIAGEN, Inc., Chatsworth, CA) and mutations were checked by Sanger ds DNA sequencing as previously described.

### Example 3

### Cloning of Mutant Protease Genes

### A. Cloning of Mutant Proteases Produced via the pMac System.

Proteases M130 and M131 can be produced by transferring the respective gene encoding either M130 or M131 from the particular *E. coli* pMc13C derivative vector into a plasmid vector which can replicate in *Bacillus.* To accomplish this, the desired mutant gene is separated from the appropriate pMc13C plasmid by digestion with the restriction endonucleases *Ava*I and *Sst*I, followed by ligation to the larger *Ava*I/*Sst*I fragment from either plasmid pH70 or pC51. These AvaI/SstI fragments from pH70 and pC51 include the DNA sequences necessary for replication in *Bacillus* and encode either kanamycin resistance (Km^{R}) or tetracycline resistance (Tc^{R}), respectively. Plasmid pH70 is constructed by cloning the ATCC 53926 alkaline protease gene carried on a *Eco*RI/*Bam*HI DNA fragment into the Km^{R} plasmid pU8110 between the *Eco*RI and *Bam*HI sites. Plasmid pC51 is constructed by cloning the ATCC 53926 protease gene carried on a *Eco*RI/*Bam*HI fragment into the Tc^{R} plasmid pBC16 between the *Eco*RI and *Bam*HI sites. The larger *Ava*I/*Sst*I fragment from either pH70 or pC51 used for cloning the DNA fragment encoding either M130 or M131 is first purified from other plasmid DNA fragments by high pressure liquid chromatography (HPLC) on an anion exchange column (Gen-Pak FAX, 4.6 mm diameter, 100 mm long; Waters, Milford, MA). Conditions for elution of the DNA are a flow rate of 0.75 ml·min⁻¹ with a gradient from 50% of Buffer A (25 mM Tris, containing 1 mM EDTA and adjusted to pH 8.0 with 2 N HCl) and Buffer B (25 mM Tris, containing 1 mM EDTA, 1 M NaCl, and adjusted to pH 8.0 with 2 N HCl) to 30% of Buffer A and 70% of Buffer B in 30 min.

The two ligated DNA's are transformed into *B. subtilis* DB104. The genes encoding the major alkaline and neutral proteases present in this strain have been inactivated (Kawamura, F. and Doi, R.A. (1984) J. Bacteriol. 160, 442-444). Cells of *B. subtilis* DB104 transformed by these plasmids grow on a nutrient-skim milk agar in the presence of either kanamycin or tetracycline. Transformants of DB104 that manufacture mutant protease are identified by the formation of clear zones of hydrolysis in the skim milk. Confirmation that the protease-producing transformants carry a plasmid-borne M130 or M131 gene with the desired mutation(s) is accomplished by purifying plasmid DNA from a culture of each transformant. The plasmid DNA is purified away from cell protein and chromosomal DNA by SDS-salt precipitation followed by chromatography over a QIAGEN ion-exchange column (QIAGEN,Inc., Chatsworth, CA). *Ava*I/*Sst*I digested plasmid DNAs from different transformants are compared with *Ava*I/*Sst*I-digested derivatives of plasmid pH70 or pC51 known to carry an intact BLAP gene. Restriction digests of these plasmids are compared by agarose gel electrophoresis to identify plasmids that have the proper-sized *Ava*I/*Sst*I DNA fragments. Selected plasmid DNAs are then sequenced across the region of the expected M130 or M131 mutations to confirm that the desired mutation(s) are present. Genes M130 and M131 cloned into the derivative of plasmid pC51 (TcR) are designated plasmids pCB56M130 and pCB56M131 respectively, while the same genes cloned into a derivative of plasmid pH70 are designated plasmids pCB76M130 and pCB76M131 respectively. One or more clones of each BLAP mutation are stored frozen in 15% glycerol at -70°C and also cultivated in shake flasks to produce mutant protease for characterization.

### B. Cloning of Mutant Proteases Produced via PCR Overlap Procedure.

As described above, the amplified PCR fragments are cloned back into plasmids pCB76M130, pCB76M131 or pCB76F49. For expression in the *B. licheniformis* production strain the AvaI/SstI fragment carrying the modified M130, M131 or F49 genes is cloned back into the pC51 type vector as described previously.

### Example 4

### Production of Mutant Proteases

Wild type BLAP protein and mutant proteins were produced by transformed *Bacillus subtilis* DB 104 in shake flasks. A hot loop was used to streak each mutant strain from a frozen cryovial culture onto an LB-skim milk agar containing either 20 µg·ml⁻¹ of kanamycin or 15 µg·ml⁻¹ of tetracycline. The plates were incubated at 37°C for 20 to 24 hours. A single, isolated colony producing a good zone of hydrolysis of the skim milk was picked into a 250 ml Erlenmeyer flask containing about 50 ml Luria Broth (LB) which contained either 20 µg·ml⁻¹ kanamycin or 15 µg·ml⁻¹ of tetracycline. The broth was incubated in a New Brunswick Series 25 Incubator Shaker at 37°C with shaking at 280 rpm for 7 to 8 hours. Either 2.5 ml of the turbid preculture was transferred into 50 ml of MLBSP containing either 20 µg·ml⁻¹ kanamycin or 15 µg·ml⁻¹ of tetracycline in each of four baffled 500 ml flasks, or 5 ml of preculture was used as an inoculum for 100 ml of MLBSP broth with antibiotic contained in each of two 500 ml baffled flasks (a 5%, v/v, transfer). All flasks were incubated at 240 rpm and 37°C for 64 hours. After 64 hours of incubation the content of a set of flasks for each culture was consolidated, transferred to 50 ml centrifuge tubes, and centrifuged at 20,000 x gₐᵥ. for 15 minutes at 4°C. The broth was filtered through Miracloth (Calbiochem Corp., #475855) into 400 ml beakers placed on ice. The pH of the solution was adjusted to 5.8 by the addition of glacial acetic acid. After 30 minutes of stirring fine debris were removed by centrifugation at 20,000 x gₐᵥ for 15 minutes. The volume of the supernatant was recorded. Aliquots of 1 ml were set aside for analysis by native and denaturing polyacrylamide gel electrophoresis (PhastSystem, Pharmacia), for determination of total proteolytic activity by the HPE method, and for active site titration. The broth was stored on ice until the protease could be purified. For long-term storage or for shipment the broth was mixed with an equal volume of propane-1,2-diol. The composition of the MLBSP media used for the production of BLAP in shake flask cultures is described in Table 3.

**TABLE 3**

| **Composition of MLBSP Media¹** | |
|---|---|
| Component | Quantity (for 1 liter of media) |
| Difco Casitone | 10 g |
| Difco Tryptone | 20 g |

| Component | Quantity (for 1 liter of media) |
|---|---|
| Difco Yeast Extract | 10 g |
| NaCl | 5 g |
| Sodium Succinate | 27 g |

| | |
|---|---|
| ¹ The pH of the media was adjusted to 7.2 with 2 N NaOH, the volume was brought to 815 ml with water, and the solution was autoclaved for 15 minutes at 121 °C at a pressure of 15 pounds per square inch. After cooling the sterile stock solutions described in Appendix 1 were added with stirring. Either a kanamycin or tetracycline stock solutions was added to the media just before use to a final concentration of 20 µg·ml⁻¹ and 15 µg·ml⁻¹, respectively. | |

### APPENDIX 1

**(Additions to MLBSP Broth)**

| Component | Quantity (for 1 liter of media) |
|---|---|
| CaCl₂.2H₂O | (30 mg·ml⁻¹ stock) 2.5 ml |
| FeSO₄.7H₂O | (1 mM stock) 0.5 ml |
| MnCl₂.4H₂O | (1 mM stock) 0.5 ml |
| Glucose | (25% (w/v) stock) 80.0 ml |
| PIPES Buffer¹ | (pH 7.2, 1 M stock) 50.0 ml |
| Phosphate Buffer² | (pH 7.0, 1.5 M stock) 50.0 ml |

| | |
|---|---|
| ¹ Piperazine-N,N'-bis(2-ethane sulfonic acid). ² A sufficient amount of 1.5 M dibasic phosphate (K₂HPO₄) was added to 200 ml of 1.5 M monobasic phosphate (KH₂PO₄) to adjust the pH to 6.0 using a pH meter (Beckman, model pH144) equipped with a combination electrode (Beckman, model #3952C). The final pH was adjusted to 7.0 with 4 M KOH. All stock solutions were autoclaved at 121°C, 15 psi for 15 min except for the FeSO₄·7H₂O which was filter sterilized using a 0.22 µm filter system (Costar 8301). | |

### Example 5

### Purification of BLAP and its Mutant Proteins

If not mentioned otherwise, all subsequent steps were performed on ice or at 4°C. Broth samples as obtained in Example 3 were filtered through a 0.2 µm filter, and the filtrate was concentrated by ultrafiltration. The retaining membrane had a nominal molecular-weight-cut-off of 10,000 mounted in a Minitan cassette (Millipore) or in a ultrafiltration cell (Amicon). For subsequent shipment or extended storage broth solutions were mixed with an equal volume of propane-1,2-diol to stabilize the protease. Otherwise, the concentrate was dialyzed for 16 hours against 20 mM sodium phosphate, pH 7.0 ('phosphate buffer'), or against 20 mM N-(2-hydroxyethyl)piperazine-N'-(2-ethanesulfonic acid) (HEPES), containing 1 mM CaCl₂ and adjusted to pH 7.8 with NaOH ('HEPES buffer'). The dialysate was clarified by centrifugation (20,000 x g_{av.} for 10 minutes) and the pH of the solution, if necessary, was re-adjusted. The samples dialyzed against phosphate buffer were equilibrated for 15 minutes with 5% (w/v) DEAE-cellulose previously equilibrated in the same buffer to trap negatively charged proteins. After removal of the DEAE-cellulose by centrifugation or filtration the supernatant was loaded onto a cation exchange column (S-Sepharose Fast Flow, Pharmacia; 16 mm diameter, 90 mm long, 18 ml bed volume, 50 ml·h⁻¹ flow rate) previously equilibrated with phosphate buffer. The samples dialyzed against HEPES buffer were applied directly to a cation exchange column (S-Sepharose Fast Flow, Pharmacia; 15 mm diameter, 75 mm long, 13 ml bed volume, 50 ml·h⁻¹ flow rate) previously equilibrated with HEPES buffer. When all colored by-products were eluted the column was washed with 3 to 5 column volumes of application buffer. Protease was eluted from the cation exchanger by including 1.0 M NaCl into phosphate buffer or 0.25 M NaCl into HEPES buffer. Fractions containing the active enzyme were pooled.

The enzyme purified in phosphate buffer was concentrated and desalted by ultrafiltration using Centricon tubes (molecular-weight-art-off 10,000; Amicon). The protein concentration was determined by the bicinchoninic acid method (BCA method, Pierce Chemical Co., Rockford, IL). For stabilization of the protease 50% (v/v) propane-1,2-diol was added to the stock solution.

The pooled fractions with enzyme purified in HEPES buffer were mixed with a 5 to 8-fold volume excess of acetone at -20°C. The protein was allowed to precipitate for 4 minutes, and the mixture was centrifuged for 4 minutes at 6,600 x gₐᵥ. The supernatant was discarded, the pellet was briefly exposed to vacuum (water aspirator) to remove most of the acetone, and the pellet was dissolved in 20 mM 2-(N-morpholino)ethanesulfonic acid (MES), containing 1 mM CaCl₂ and adjusted to pH 5.8 with 2 N NaOH, to give an approximate protein concentration of 30 mg·ml⁻¹. The solution was clarified by centrifugation in an Eppendorf centrifuge for 3 minutes at full speed (13,000 x gₘₐₓ) and stored frozen until used. The protein concentration was determined by the biuret method (Gornall, A.G., Bardawill, C.S., and David, M.M. (1948) J. Biol. Chem. 177, 751-766).

### Example 6

### Determination of Active Protease

The active enzyme concentration was determined by active site titration with the inhibitor phenylmethyl sulfonylfluoride. A protease solution was prepared in 10 mM sodium phosphate, pH 6.5, at an approximate concentration of 100 µM based on protein determination. Inhibitor concentrations were equivalent to an estimated molar ratio of 0.25, 0.5, 0.75, 1.0 and 1.25. The mixtures were allowed to react for one hour at room temperature. Residual enzyme activity was measured spectrophotometrically by the AAPF-pNA method (see below).

### Example 7

### Determination of Proteolytic Activity

Two different protease assays were used. With the HPE method protease activity was established at a single concentration of casein as substrate. In the AAPF-pNA assay initial rates of succinyl-L-alanyl-L-alanyl-L-prolyi-L-phenylalanyl-*p*-nitroanilide (AAPF-pNA; Bachem Bioscience, Philadelphia, PA) supported catalysis were used to determine the kinetic parameters Kₘ, k_{cat}, and k_{cat}/Kₘ.

### A. Protease Assay by the HPE Method

Proteolytic activity was determined by a discontinuous assay using casein as a substrate. The final concentrations of the substrate solution were 12 mg.ml⁻¹ of casein (prepared according to Hammarsten; Merck, Darmstadt, #2242) and 30 mM Tris in synthetic tap water. Synthetic tap water is a solution of 0.029% (w/v) CaCl₂·2H₂O, 0.014% (w/v) MgCl₂·6H₂O, and 0.021 % (w/v) NaHCO₃ with a hardness of 15°dH (deutsche Härte. German hardness). The substrate solution is heated to 70°C and pH is adjusted to 8.5 at 50°C using 0.1 N NaOH. The protease solution is prepared with 2% (w/v) anhydrous pentasodium tripolyphosphate in synthetic tap water, adjusted to pH 8.5 with hydrochloric acid. To 600 µl of casein substrate solution 200 µl of enzyme solution is added. The mixture is incubated at 50°C for 15 minutes. The reaction is terminated by the addition of 600 µl of 0.44 M trichloroacetic acid (TCA), 0.22 M sodium acetate in 3% (v/v) glacial acetic acid. After cooling on ice for 15 minutes the TCA-insoluble protein is removed by centrifugation, an aliquot of 900 µl is mixed with 300 µl of 2N NaOH and the absorbance of this mixture containing TCA-soluble peptides is recorded at 290 nm. Control values are produced by adding 600 µl of TCA solution to 600 µl of casein solution followed by 200 µl of enzyme solution.

A protease solution which produces under the conditions of this assay an absorbance change of 0.500 OD at 290 nm is declared to have an activity 10 HPE per ml. Data for BLAP and BLAP mutant proteins are summarized below (Table 4).

### B. Protease Assay With the Chromogenic Substrate AAPF-pNA

Protease samples were diluted with 50% (v/v) 1,2-propanediol in 100 mM Tris, adjusted with 2 N HCl to pH 8.6 at 25°C ('Tris-propanediol buffer'), in which they were stable for at least 6 h at room temperature. A stock solution of 160 mM AAPF-pNA was prepared in dimethylsulfoxide dried over molecular sieve beads (Aldrich; 4 A, 4-8 mesh) for at least 24 h prior to use. Fixed point assays were performed at 25°C with 1.6 mM AAPF-pNA in 100 mM Tris, adjusted with 2 N HCl to pH 8.6 at 25°C, in a total volume of 1.020 ml. The substrate was added to the assay buffer 1 minute prior to the assay initiation and the reaction was started by addition of enzyme at a final concentration of 20 ng to 1.3 µg of protein per ml (0.75 to 48.5 nM enzyme) depending on specific activity. Release of *p*-nitroanilide was monitored at 410 nm, and a molar extinction coefficient of 8,480 M⁻¹cm⁻¹ was used to calculate amount and concentration of product formed (DelMar, E.G., Largman, C., Brodrick, J.W., and Geokas, M.C. (1979) Anal Biochem. 99, 316-320).

Kinetic parameters were calculated from a velocity vs. substrate concentration plot constructed from initial rates measured once each at 12 different AAPF-pNA concentrations ranging from 0.16 to 3.2 mM. Data were fitted to a hyperbolic curve and proportionally weighted using the program ENZFITTER (Leatherbarrow, R.J. (1987) ENZFITTER. Biosoft, Cambridge, UK). A nominal molecular weight of 26.8 kDa was used in all calculations that required the interconversion of protein concentration and molarity of protease enzyme (Table 4).

**TABLE 4**

| **Kinetic Data of BLAP and BLAP Mutant Proteases** | | | | |
|---|---|---|---|---|
| Label | Activity (HPE-mg⁻¹) | (s⁻¹) | (mM) | (s⁻¹·mM⁻¹) |
| M131 | 3670 | 153 | 0.98 | 156 |
| F11 | 3760 | 174 | 1.88 | 92 |
| F43 | 3780 | 171 | 1.73 | 99 |
| F44 | 3750 | 257 | 2.49 | 103 |
| F45 | 3710 | 208 | 3.05 | 68 |
| F46 | 3180 | 358 | 2.0 | 179 |
| F47 | 3610 | 249 | 2.0 | 124 |
| F49 | 4610 | 38 | 3.5 | 11 |
| F54 | 2510 | 7 | 5.5 | 1.3 |
| F55 | 3200 | 11 | 4.3 | 2.6 |

### Example 8

### Protein Structure Verification

Mutations introduced into the BLAP were verified by DNA sequence analysis through the immediate vicinity of the point of mutation. However, spontaneous mutations outside the region of site-directed mutagenesis are known to occur and it was imperative to establish that the determined property of a mutant protease indeed resides with the amino acid sequence deduced from the nucleotide sequence. Therefore, a tryptic map of BLAP was produced which was verified by amino acid sequence analysis of the individual peaks and to which all mutant BLAP proteins were compared. Not only in peptides with ≤ 17 amino acid residues, but also in peptides 5T, 7T, and 10T with 48, 44, and 49 amino acid residues, a single (even conservative) substitution resulted in a significant change in retention time under the conditions of the separation. Particularly close calls were resolved by co-digestion of equal quantities of the reference (BLAP) and the mutant protein.

An aliquot of up to 5 mg of protease from a stock solution was placed on ice in a 2.2-ml Eppendorf tube, then mixed with 1.0 ml of 0.15 N HCl and water (both chilled) to give a final concentration of 3.33 mg· ml⁻¹ protein and 0.1 N HCl in a total volume of 1.5 ml. After the mixture had been incubated for 30 minutes, protein was precipitated by the addition of 165 µl of chilled 50% (w/v) trichloroacetic acid (TCA). The precipitate was allowed to stand on ice for 5 minutes and then pelleted by centrifugation for 4 minutes at 13,000 x gₘₐₓ (Eppendorf centrifuge). The pellet was washed once with 1 ml of 80% (v/v) acetone and briefly dried *in vacuo.*

All reagent solutions and water needed for the tryptic digest were passed through a 0.45 µm filter (Ultrafree-MC, Millipore Products) prior to use. The pellet of the denatured protein (5 mg; 185 nmol) was dissolved in 90 µl of 0.8 M ammonium bicarbonate, containing 8 M urea. This solution was slowly diluted with 360 µl of water and then passed by centrifugation through a 0.45 µm filter. Subsequent steps were carried out in 0.5-ml siliconized microtubes (Phenix Res. Products). An aliquot of 300 µl was mixed with 13 µl of 2.5 mg·ml⁻¹ trypsin in 1 mM HCl (mass ratio of BLAP:trypsin = 100:1). For a control, 100 µl of the protein solution was mixed with 4.5 µl of 1 mM HCl. The remaining 50 µl aliquot of protein solution was mixed with 5 µl of 10% (v/v) trifluoroacetic acid (TFA) and used as control of the starting material. The two other solutions were incubated for 10 minutes at 37°C. The reactions were terminated by adding 30 µl and 10 µl of 10% (v/v) TFA to the digest and the control, respectively. The peptide mixture was separated by reverse-phase HPLC.

The HPLC equipment was from Waters and consisted of an autosampler (model 715 Ultra Wisp), a dual pump system (model 600E) and a diode array detector (model 990). Sampling and gradient formation was governed by Waters' software program '990* Powerline'. Tryptic peptides were separated on a C₁₈ column (Vydac model 218TP54; 4.6 x 250 mm; 5 µ particle size; 300 A pore size). In line with the separation column was a C₁₈ guard column (Vydac model 218FSK104, 10µ particle size). Separation column and guard column were housed in a column heater set to 30±1 °C. The solvent system used was: Solvent A = 0.1 % (v/v) TFA in water; Solvent B = 0.08% (v/v) TFA in acetonitrile. After sample loading the C₁₈ column was developed for 3 minutes with Solvent A followed by a gradient from 0 to 35% (v/v) of Solvent B in Solvent A in 70 minutes. At 70 minutes the gradient increased to 100% Solvent B in 15 minutes and then returned to 100% Solvent A in 15 minutes. Prior to the next injection, the column was equilibrated for at least 20 minutes with Solvent A.

Absorbance changes were recorded at 215 nm and at 280 nm. Quantities of peptides mixtures separated for analytical and preparative purposes ranged from 11 µg (0.4 nmol) to 500 µg (18 nmol) in a volume of 5 to 50 µl at concentrations from 2.2 to 10 mg·ml⁻¹.

For peptide sequencing the eluate was hand-fractionated according to absorbance changes observed on the attached recorder. Previous studies with β-mercaptoethanol showed that the delay time from the time of recording to the time of elution from the system was less than 2 seconds. Fractions were concentrated *in vacuo* in a Speed Vac Concentrator (Savant, Hicksville, NY) to less than 100 µl and brought to 100 µl with aqueous TFA to achieve a final TFA concentration of 1%. Care was taken that during concentration the samples did not reach complete dryness.

### Example 9

### Characterization of the Proteases by Tenside Stability

Stability of the protease mutants to tensides was tested with SDS as typical anionic detergent. Stability was tested in 50 mM sodium carbonate, pH 10.5 at 50°C, containing 1% (w/v) SDS. Protease proteins were incubated at a final protein concentration of 0.25 mg·ml⁻¹. Periodically, an aliquot was removed from the incubation mixture and diluted into Tris-propanediol buffer chilled on ice. Residual protease activity was determined by the AAPF-pNA assay at a substrate concentration of 1.1 mM. Stability is expressed as half-life (t_{1/2}) of activity determined from semi-logarithmic plots of residual activity as function of time.

### Example 10

### Wash Performance of the Protease Mutants

The wash performance was tested in a specially developed washing test using cotton swatches soiled with egg and soot (ER) and with blood, milk and soot (BMR). The washing tests were performed in an Atlas launderometer (type LP 2), equipped with stainless steel test vessels each containing a defined detergent composition plus the protease to be tested.

The pre-washed cotton was soiled with a defined amount of soil and air-dried for 6 days. The launderometer beakers were filled with 1 swatch of soiled and 3 swatches of unsoiled cotton. Ten metal balls (10 mm diameter) were added for mechanical treatment. The washing time was 30 minutes with a final temperature of 30°C reached after 4 minutes of heating.

Laundry detergents for these tests were of typical composition for European usage (Jakobi, G. and Löhr, A. (1987) Detergents and Textile Washing, VCH, Weinheim, Germany). The concentrations of a delicate fabric detergent for easy-care and colored fabrics (5-15% (w/w) anionic surfactants, 1-5% (w/w) nonionic surfactants), a heavy duty compact detergent (8% (w/w) anionic surfactants, 6% (w/w) nonionic surfactants, with tetraacetylethylenediamine (TAED) and perborate bleach) and a super compact detergent concentrate (18% (w/w) anionic surfactants, 2.5% (w/w) nonionic surfactants, with TAED and perborate bleach) were 0.5 g, 0.5 g, and 0.4 g, respectively, in 100 ml of water at 16°dH (deutsche Härte, German hardness). The super compact detergent is an extruded detergent granulate, described by a number of patent applications (WO 91/02047, WO 91/13678, WO 93/02176, WO 93/15180, WO 94/01526). In all cases the pH was 10.4. A protease was added to washing solutions on the basis of its enzymatic activity measured in HPE at a ratio of 0, 50, 100, 200, 300, 400, 500, 700 and 1000 HPE per gram of detergent.

Subsequent to washing, the fabric was rinsed with running tap water, air-dried and ironed. The enzymatic washing effect was determined by the change (ΔRem) of the remission (%Rem) at 440 nm (400-500 nm) measured on a Dr. Lange color difference measuring instrument (Micro Color). ΔRem is the difference in remission after washing with added protease and the remission after washing without added protease. Results from the wash performance tests are presented in Tables 5 and 6.

The improvement in washing performance was determined by the ratio of wild type enzyme necessary to achieve a standard ΔRem, versus the amount of mutant enzyme to achieve an identical effect. Thus an improvement of 2 indicates that half of the mutant enzyme is needed to get the same effect as with the wild type enzyme.

**TABLE 5**

| **Washing Effect of BLAP and BLAP Mutant Proteins on Blood-Milk-Soot-Stains** | | | |
|---|---|---|---|
| Enzyme | Detergent | | |
| | **A¹** | **B** | **C** |
| | Improvement Ratio² | Improvement Ratio | Improvement Ratio |
| BLAP | 1 | 1 | 1 |
| M131 | 1 | 1 | 1 |
| F43 | 2.8 | 1.8 | 0.7 |
| F44 | 2.0 | 1.8 | <0.7 |
| F45 | 2.6 | 1.1 | <0.7 |
| F46 | 2.9 | 1.9 | 1 |
| F47 | 1.5 | 1.8 | 0.8 |
| F49 | 1.8 | 1 | 2.5 |

| | | | |
|---|---|---|---|
| ¹ Detergent **A** is a Heavy Duty Compact Detergent, Detergent **B** is a Heavy Duty Super Compact Detergent and Detergent **C** is a Delicate Fabric Detergent. ² For definition see text. | | | |

**TABLE 6**

| **Washing Effect of BLAP and BLAP Mutant Proteins on Egg-Soot-Stains** | | |
|---|---|---|
| Enzyme | Detergent | |
| | Heavy Duty Compact Detergent | Heavy Duty Super Compact Detergent |
| | Improvement Ratio¹ | Improvement Ratio |
| BLAP | 1 | 1 |
| M131 | 1 | 1 |
| F46 | 1.5 | 1 |
| F47 | 1 | 1 |
| F49 | 4.0 | 1.7 |

| | | |
|---|---|---|
| ¹ For definition see text. | | |

### Example 11

An enzymatic detergent composition is prepared by mixing about 30 wt% of a concentrated preparation of a protease mutant as defined in claim 6, with about 5 wt% of cellulose, 5 wt% of saccharose, 20 wt% of wheat flour, 30 wt% of starch, 5 wt% of carboxymethylcellulose and 5% of polyethylene glycol (mw 20,000). The resulting mixture is granulated by extrusion granulation. After drying, the granulate has an activity of 70,000 to 250,000 HPE/g. The granulate is coated with polyethylene glycol (mw 6000) containing TiO₂. The granulated protease enzyme is then mixed with 100g Heavy Duty Compact Detergent (Porsil supra) containing sodium perborate\TAED resulting in a standard protease activity of 1200 HPE/g.

### Example 12

An enzymatic detergent composition is prepared according to Example 11 except the protease is replaced by the protease as defined in claim 7.

### Example 13

A liquid enzymatic detergent composition is prepared by mixing together at room temperature about 13 wt% C10-C13 linear alkylbenzene-sulfonic acid, about 5 wt% alkylpolyglycoside(C12-C14), about 10 wt% of a C13 alcohol polyethoxylate having 7 EO units, about 6 wt% lauric acid, about 7 wt% of oleic acid, about 5 wt% triethanolamine, about 5 wt% propanediol 1,2, about 2 wt sodium hydroxide, about 1 wt% citric acid, about 7 wt% ethanol, about 1 wt% citric acid about 7 wt% ethanol, about 1-hydroxyethane-1,1-diphosphonic acid and the remainder being water.

The pH of the resulting solution is 8.1 (measured as a 10% aqueous solution). A sufficient amount of the protease as defined in claim 6 is added to yield a composition having about 0.5 wt% of liquid protease concentrate (1250 HPE/g).

### Example 14:

The KC ratio of protease F49 and, for comparison, the KC ratios of three commercial proteases were determined by the method described above. The following results were obtained:

| Protease | KC ratio |
|---|---|
| MAXACALV®^{a} | 0.92 |
| SAVINASEV®^{b} | 0.80 |
| BLAP® | 0.83 |
| F49 | 0.63 |

a) Manufacturer: Gist Brocades, Netherlands
b) Manufacturer: Novo Nordisk, Denmark

### Example 15

1 g of wool yarn was added to 50 ml of an F 49-containing sodium carbonate buffer solution (pH 10.5) with a proteolytic activity of 5.6 PU/ml and the whole was left to react for 1 hour at room temperature. The quantity of amino acids released and peptides was then determined with TNBS (control determination in buffer solution without protease). The wool damage value obtained under these conditions for the protease BLAP was put at 100%. Under these conditions, the protease F 49 had a value of 31%.

### Example 16

The test described in Example 2 was repeated using 1 g of silk yarn. The following silk damage values were obtained:

| Protease | Silk damage |
|---|---|
| BLAP | 100% |
| ESPERASE®^{a} | 165% |
| SAVINASE®^{a} | 89% |
| F 49 | 28% |
| a) Manufacturer: Novo Nordisk, Denmark | |

### Example 17

In a MIELE® Electronic W 793 washing machine, articles of clothing of wool were washed 9 times at 40°C (water hardness 16°d, liquor ratio 1:15) in the presence of ballast washing of cotton (total load 1.2 kg). 98 g of an enzyme-free coloreds detergent (control test), to which 49 ml of a BLAP 140 solution (comparison test) or an equally active quantity, expressed in PU of F 49 (invention) had been added, were used in the wash liquor. The washed woollen fabrics were dried and pieces of specific dimensions were cut out and weighed. The reduction in weight, expressed in % of the value before washing, is shown in the following Table:
a) Blend of 80% Merino wool and 20% polyamide

### Example 18

Example 4 was repeated using grey socks of pure wool, the proteases again being used in quantities of equal activity (1080 PU/g of the detergent); in contrast to Example 4, 5 washes were carried out at 60°C. The reduction in weight was only 10.7% where the protease F 49 was used and 40.5% where BLAP® was used.

### Example 19

A solution containing 12 g/l of the substrate (casein or keratin) and 30 mM of sodium tripolyphosphate in water with a hardness of 15°dH (containing 0.058% by weight of CaCl₂·2H₂O, 0.028% by weight of MgCl₂·6H₂O and 0.042% by weight of NaHCO₃) is heated to 70°C and the pH is adjusted to a value of 8.5 by addition of 0.1 N NaOH at 50°C 200 µl of a solution of the enzyme to be tested in 2% by weight sodium tripolyphosphate buffer solution (pH 8.5) are added to 600 µl of the substrate solution. The reaction mixture is incubated for 15 minutes at 50°C. The reaction is then terminated by addition of 500 µl of TCA solution (0.44 M trichloroacetic acid and 0.22 M sodium acetate in 3% by volume acetic acid) and cooling (ice bath at 0°C, 15 minutes). The protein insoluble in TCA is removed by centrifugation and 900 µl of the supernatant phase are diluted with 300 µl of 2 N NaOH. The absorption of this solution at 290 nm is determined with an absorption spectrometer, the absorption zero value having to be determined by measurement of a centrifuged solution prepared by mixing 600 µl of the above-mentioned TCA solution with 600 µl of the above-mentioned substrate solution and subsequent addition of the enzyme solution.

### Example 20

Pre-washed cotton fabrics were soiled with equal amounts of soil and air dried for 3 days. Launderometer beakers were filled with 6 swatches of soiled and unsoiled cotton and 10 metal balls (1 cm in diameter) and washed in an Atlas LP-2 type laundrometer for 30 minutes with a final temperature of 30°C, which was reached after 4 minutes of heating. A fine specialty detergent formulation was used for easy-care and colored fabrics (0.75 g/100 ml). A heavy duty compact detergent (0.5 g/100 ml) and a super compact detergent concentrate (0.4 g/100 ml) were also used. The composition of the detergents is described in "Detergents and Textile Washing", G. Jacobi & A. Löhr, VCH, Weinheim, ISBN 0-89573-687-X, 1987. The water used was 16° dH (German Hardness) and the pH was 4. Enzyme activities of the detergents were 0, 50, 100, 200, 300, 400, 500, 700, and 1000 HPE/g detergent. After washing, the test swatches were rinsed in tap water, air dried, and ironed. The enzymatic washing effect was determined by the change (ΔRem) in the remission (%Rem) at 440 nm measured using a Dr. Lange color difference measuring instrument Micro Color, ΔRem being the difference in remission after wash with protease added and the remission after wash without protease. The results of washing performance for various enzyme-containing detergents and detergent compositions containing a combination of enzymes are given in Tables 2 and 3 below. Each entry is a ratio of the amount of mutant enzyme achieving a standard ΔRem, versus the amount of the wild-type enzyme to achieve an identical cleaning effect. A entry of 2 indicates that twice as much of the wild-type enzyme was needed as a particular mutant to achieve the same cleaning effect. The data also show that a detergent composition containing a combination of mutants F46 and F49 produced a greater cleaning effect than the average of the individual enzymes.

**Table 2**

| | Washing Effect of Enzymes on Blood-Milk-Soot Stains Detergent | |
|---|---|---|
| Enzyme | Heavy Duty | Heavy Duty Compact |
| BLAP/M131 | 1 | 1 |
| F46 | 2.9 | 1.9 |
| F49 | 1.8 | 1.0 |
| F46+F49 | 2.6 | 2.2 |

**Table 3**

| | Washing Effect of Enzymes on Egg-Soot Stains Detergent | |
|---|---|---|
| Enzyme | HeavyDuty | Heavy Duty Compact |
| BLAP/M131 | 1 | 1 |
| F46 | 1.5 | 1.3 |
| F49 | 4.0 | 1.7 |
| F46+F49 | 3.3 | 2.2 |

### Example 21

A similar composition as described in example 13 is prepared except the protease is replaced by the protease as defined in claim 7.

### SEQUENCE LISTING

(1) GENERAL INFORMATION:
   (i) APPLICANT: Wilson, Charles R
   (ii) TITLE OF INVENTION: Proteases with Improved Wash Performance
   (iii) NUMBER OF SEQUENCES: 18
   (iv) CORRESPONDENCE ADDRESS:
      (A) ADDRESSEE: Henkel Corporation
      (B) STREET: 140 Germantown Pike, Suite 150
      (C) CITY: Plymouth Meeting
      (D) STATE: Pennsylvania
      (E) COUNTRY: USA
      (F) ZIP: 19462
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Drach, John E.
      (B) REGISTRATION NUMBER: 32891
      (C) REFERENCE/DOCKET NUMBER: H0587
   (ix) TELECOMMUNICATION INFORMATION:
      (A) TELEPHONE: 215-832-2215
      (B) TELEFAX: 215-941-6067
(2) INFORMATION FOR SEQ ID NO:1:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, R99S, A188P, V193M, V199I
   (xi) SEQUENCE DESCRIPTION: SEQ ID NO:1:
   GCGCAAACAG TGCCATGGGG AATTAGCCGT GTGCAAGCCC CGGCTGCCCA TAACCGTGGA   60
   TTGACAGGTT CTGGTGTAAA AGTTGCTGTC CTCGATACAG GTATTTCCAC TCATCCAGAC  120
   TTAAATATTC GTGGTGGCGC TAGCTTTGTA CCAGGGGAAC CATCCACTCA AGATGGGAAT  180
   GGGCATGGCA CGCATGTGGC CGGGACGATT GCTGCTTTAA ACAATTCGAT TGGCGTTCTT  240
   GGCGTAGCGC CTAGTGCGGA ACTATACGCT GTTAAAGTTT TAGGAGCCGA CGGTAGCGGT  300
   GCAATCAGCT CGATTGCCCA AGGGTTGGAA TGGGCAGGGA ACAATGGCAT GCACGTTGCT  360
   AATTTGAGTT TAGGAAGCCC TTCGCCAAGT GCCACACTTG AGCAAGCTGT TAATAGCGCG  420
   ACTTCTAGAG GCGTTCTTGT TGTAGCGGCA TCTGGGAATT CAGGTGCAAG CTCAATCAGC  480
   TATCCGGCCC GTTATGCGAA CGCAATGGCA GTCGGAGCTA CTGACCAAAA CAACAACCGC  540
   GCCAGCTTTT CACAGTATGG CCCAGGGCTT GACATTATGG CACCAGGGGT AAACATTCAG  600
   AGCACATACC CAGGTTCAAC GTATGCCAGC TTAAACGGTA CATCGATGGC TACTCCTCAT  660
   GTTGCAGGTG CAGCAGCCCT TGTTAAACAA AAGAACCCAT CTTGGTCCAA TGTACAAATC  720
   CGCAACCATC TAAAGAATAC GGCAACGAGC TTAGGAAGCA CGAACTTGTA TGGAAGCGGA  780
   CTTGTCAATG CAGAAGCGGC AACACGC                                       807
(2) INFORMATION FOR SEQ ID NO:2:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, R99G, A188P, V193M, V1991
   (xi) SEQUENCE DESCRIPTION: SEQ ID NO:2:
   GCGCAAACAA TCCCATGGGG AATTAGCCGT GTGCAAGCCC CGGCTGCCCA TAACCGTGGA   60
   TTGACAGGTT CTGGTGTAAA AGTTGCTGTC CTCGATACAG GTATTTCCAC TCATCCAGAC  120
   TTAAATATTC GTGGTGGCGC TAGCTTTGTA CCAGGGGAAC CATCCACTCA AGATGGGAAT  180
   GGGCATGGCA CGCATGTGGC CGGGACGATT GCTGCTTTAA ACAATTCGAT TGGCGTTCTT  240
   GGCGTAGCGC CTAGTGCGGA ACTATACGCT GTTAAAGTTT TAGGAGCCGA CGGTGGAGGT  300
   GCAATCAGCT CGATTGCCCA AGGGTTGGAA TGGGCAGGGA ACAATGGCAT GCACGTTGCT  360
   AATTTGAGTT TAGGAAGCCC TTCGCCAAGT GCCACACTTG AGCAAGCTGT TAATAGCGCG  420
   ACTTCTAGAG GCGTTCTTGT TGTAGCGGCA TCTGGGAATT CAGGTGCAAG CTCAATCAGC  480
   TATCCGGCCC GTTATGCGAA CGCAATGGCA GTCGGAGCTA CTGACCAAAA CAACAACCGC  540
   GCCAGCTTTT CACAGTATGG CCCAGGGCTT GACATTATGG CACCAGGGGT AAACATTCAG  600
   AGCACATACC CAGGTTCAAC GTATGCCAGC TTAAACGGTA CATCGATGGC TACTCCTCAT  660
   GTTGCAGGTG CAGCAGCCCT TGTTAAACAA AAGAACCCAT CTTGGTCCAA TGTACAAATC  720
   CGCAACCATC TAAAGAATAC GGCAACGAGC TTAGGAAGCA CGAACTTGTA TGGAAGCGGA  780
   CTTGTCAATG CAGAAGCGGC AACACGC                                       807
(2) INFORMATION FOR SEQ ID NO:3:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE: (B) STRAIN: Bacillus lentus DSM 5483
   (vii) IMMEDIATE SOURCE:
      (B) CLONE: S3T, V41, R99A, A188P, V193M, V1991
   (xi) SEQUENCE DESCRIPTION: SEQ ID NO:3:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTGCAGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATT CAGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC TTAAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:4:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, R99S, A188P, V193M, V1991
   (xi) SEQUENCE DESCRIPTION: SEQ ID NO:4:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTAGCGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATT CAGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC TTAAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:5:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, S154E, A188P, V193M, V1991
   (xi) SEQUENCE DESCRIPTION: SEQ ID NO:5:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTAGAGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATG AAGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC TTAAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:6:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, S154D, A188P, V193M, V1991
   (xi) SEQUENCE DESCRIPTION: SEQ ID NO:6:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTAGAGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATG ACGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC TTAAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:7:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, A188P, V193M, V1991, L211D
   (xi) SEQUENCE DESCRIPTION: SEQ ID NO:7:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTAGAGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATT CAGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC GACAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:8:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, R99G, A188P, V193M, V1991, L211D
   (xi) SEQUENCE DESCRIPTION: SEQ ID NO:8:
   GCGCAAACAA TCCCATGGGG AATTAGCCGT GTGCAAGCCC CGGCTGCCCA TAACCGTGGA 60
   TTGACAGGTT CTGGTGTAAA AGTTGCTGTC CTCGATACAG GTATTTCCAC TCATCCAGAC 120
   TTAAATATTC GTGGTGGCGC TAGCTTTGTA CCAGGGGAAC CATCCACTCA AGATGGGAAT 180
   GGGCATGGCA CGCATGTGGC CGGGACGATT GCTGCTTTAA ACAATTCGAT TGGCGTTCTT 240
   GGCGTAGCGC CTAGTGCGGA ACTATACGCT GTTAAAGTTT TAGGAGCCGA CGGTGGAGGT 300
   GCAATCAGCT CGATTGCCCA AGGGTTGGAA TGGGCAGGGA ACAATGGCAT GCACGTTGCT 360
   AATTTGAGTT TAGGAAGCCC TTCGCCAAGT GCCACACTTG AGCAAGCTGT TAATAGCGCG 420
   ACTTCTAGAG GCGTTCTTGT TGTAGCGGCA TCTGGGAATT CAGGTGCAAG CTCAATCAGC 480
   TATCCGGCCC GTTATGCGAA CGCAATGGCA GTCGGAGCTA CTGACCAAAA CAACAACCGC 540
   GCCAGCTTTT CACAGTATGG CCCAGGGCTT GACATTATGG CACCAGGGGT AAACATTCAG 600
   AGCACATACC CAGGTTCAAC GTATGCCAGC GACAACGGTA CATCGATGGC TACTCCTCAT 660
   GTTGCAGGTG CAGCAGCCCT TGTTAAACAA AAGAACCCAT CTTGGTCCAA TGTACAAATC 720
   CGCAACCATC TAAAGAATAC GGCAACGAGC TTAGGAAGCA CGAACTTGTA TGGAAGCGGA 780
   CTTGTCAATG CAGAAGCGGC AACACGC 807
(2) INFORMATION FOR SEQ ID NO:9:
   (i) SEQUENCE CHARACTERISTICS:
      (A) LENGTH: 807 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (B) STRAIN: Bacillus lentus DSM 5483
   (vii) IMMEDIATE SOURCE:
      (B) CLONE: S3T, V41, S154E, A188P, V193M, V1991, L211D
   (xi) SEQUENCE DESCRIPTION: SEQ ID NO:9:
   GCGCAAACAA TCCCATGGGG AATTAGCCGT GTGCAAGCCC CGGCTGCCCA TAACCGTGGA   60
   TTGACAGGTT CTGGTGTAAA AGTTGCTGTC CTCGATACAG GTATTTCCAC TCATCCAGAC  120
   TTAAATATTC GTGGTGGCGC TAGCTTTGTA CCAGGGGAAC CATCCACTCA AGATGGGAAT  180
   GGGCATGGCA CGCATGTGGC CGGGACGATT GCTGCTTTAA ACAATTCGAT TGGCGTTCTT  240
   GGCGTAGCGC CTAGTGCGGA ACTATACGCT GTTAAAGTTT TAGGAGCCGA CGGTAGAGGT  300
   GCAATCAGCT CGATTGCCCA AGGGTTGGAA TGGGCAGGGA ACAATGGCAT GCACGTTGCT  360
   AATTTGAGTT TAGGAAGCCC TTCGCCAAGT GCCACACTTG AGCAAGCTGT TAATAGCGCG  420
   ACTTCTAGAG GCGTTCTTGT TGTAGCGGCA TCTGGGAATG AAGGTGCAAG CTCAATCAGC  480
   TATCCGGCCC GTTATGCGAA CGCAATGGCA GTCGGAGCTA CTGACCAAAA CAACAACCGC  540
   GCCAGCTTTT CACAGTATGG CCCAGGGCTT GACATTATGG CACCAGGGGT AAACATTCAG  600
   AGCACATACC CAGGTTCAAC GTATGCCAGC GACAACGGTA CATCGATGGC TACTCCTCAT  660
   GTTGCAGGTG CAGCAGCCCT TGTTAAACAA AAGAACCCAT CTTGGTCCAA TGTACAAATC  720
   CGCAACCATC TAAAGAATAC GGCAACGAGC TTAGGAAGCA CGAACTTGTA TGGAAGCGGA  780
   CTTGTCAATG CAGAAGCGGC AACACGC                                      807
(2) INFORMATION FOR SEQ ID NO:10:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F11
   (xi) SEQUENCE DESCRIPTION: SEQ ID NO:10:
   Ala Gln Thr Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Ser Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:11:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F43
   (xi) SEQUENCE DESCRIPTION: SEQ ID NO:11:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Gly Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:12:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F44
   (xi) SEQUENCE DESCRIPTION: SEQ ID NO:12:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Ala Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:13:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F45
   (xi) SEQUENCE DESCRIPTION: SEQ ID NO:13:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Ser Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:14:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F46
   (xi) SEQUENCE DESCRIPTION: SEQ ID NO:14:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Glu Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:15:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F47
   (xi) SEQUENCE DESCRIPTION: SEQ ID NO:15:
(2) INFORMATION FOR SEQ ID NO:16:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F49
   (xi) SEQUENCE DESCRIPTION: SEQ ID NO:16:
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Asp Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:17:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F54
   (xi) SEQUENCE DESCRIPTION: SEQ ID NO:17:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Gly Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Asp Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
(2) INFORMATION FOR SEQ ID NO:18:
   (i) SEQUENCE CHARACTERISTICS:
      (A) LENGTH: 269 amino acids
      (B) TYPE: amino acid
      (D) TOPOLOGY: unknown
   (ii) MOLECULE TYPE: protein
   (iii) HYPOTHETICAL: NO
   (iv) ANTI-SENSE: NO
   (vi) ORIGINAL SOURCE:
      (A) ORGANISM: Serine Protease
      (B) STRAIN: Bacillus lentus DSM 5843
   (vii) IMMEDIATE SOURCE:
      (B) CLONE: F55
   (xi) SEQUENCE DESCRIPTION: SEQ ID NO:18:
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Glu Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Asp Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265

### SEQUENCE LISTING

<110> Henkel Kommanditgesellschaft auf Aktien
<120> Improved enzymes and detergents containing them
<130> H 587/876/939 PCT/EP
<140> 95912559.2
   <141> 1995-02-23
<150> US 08/201120
   <151> 1994-02-24
<150> US 08/373818
   <151> 1995-01-17
<160> 26
<170> PatentIn Ver. 2.1
<210> 1
   <211> 807
   <212> DNA
   <213> Bacillus lentus DSM 5483 clone F11
<400> 1
   gcgcaaacag tgccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagcggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 2
   <211> 807
   <212> DNA
   <213> Bacillus lentus DSM 5483 clone F43
<400> 2
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtggaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 3
   <211> 807
   <212> DNA
   <213> Bacillus lentus DSM 5483 clone F44
<400> 3
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtgcaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 4
   <211> 807
   <212> DNA
   <213> Bacillus lentus DSM 5483 clone F45
<400> 4
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagcggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 5
   <211> 807
   <212> DNA
   <213> Bacillus lentus DSM 5483 clone F46
<400> 5
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatg aaggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc                                     807
<210> 6
   <211> 807
   <212> DNA
   <213> Bacillus lentus DSM 5483 clone F47
<400> 6
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatg acggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc                                     807
<210> 7
   <211> 807
   <212> DNA
   <213> Bacillus lentus DSM 5483 clone F49
<400> 7
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc gacaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 8
   <211> 807
   <212> DNA
   <213> Bacillus lentus DSM 5483 clone F54
<400> 8
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtggaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc gacaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 9
   <211> 807
   <212> DNA
   <213> Bacillus lentus DSM 5483 clone F55
<400> 9
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatg aaggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc gacaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc                                     807
<210> 10
   <211> 269
   <212> PRT
   <213> Bacillus lentus DSM 5483 clone F11
<400> 10
   Ala Gln Thr Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
   85 90 95
   Asp Gly Ser Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 11
   <211> 269
   <212> PRT
   <213> Bacillus lentus DSM 5483 clone F43
<400> 11
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Gly Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Pro Gly Leu Asp Ile
               180                 185                 190
   Met Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 12
<211> 269
   <212> PRT
   <213> Bacillus lentus DSM 5483 clone F44
<400> 12
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Ala Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Pro Gly Leu Asp Ile
               180                 185                 190
   Met Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 13
   <211> 269
   <212> PRT
   <213> Bacillus lentus DSM 5483 clone F45
<400> 13
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Ser Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 14
   <211> 269
   <212> PRT
   <213> Bacillus lentus DSM 5483 clone F46
<400> 14
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Glu Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 15
   <211> 269
   <212> PRT
   <213> Bacillus lentus DSM 5483 clone F47
<400> 15
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Asp Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 16
   <211> 269
   <212> PRT
   <213> Bacillus lentus DSM 5483 clone F49
<400> 16
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Asp Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 17
   <211> 269
   <212> PRT
   <213> Bacillus lentus DSM 5483 clone F54
<400> 17
   Ala Gln Thr Ile Pro Trp Gly Ile Ser Arg Val Gln Ala Pro Ala Ala
    1               5                  10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Gly Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Pro Gly Leu Asp Ile
               180                 185                 190
   Met Ala Pro Gly Val Asn Ile Gln Ser Thr Tyr Pro Gly Ser Thr Tyr
           195                 200                 205
   Ala Ser Asp Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 18
   <211> 269
   <212> PRT
   <213> Bacillus lentus DSM 5483 clone F55
<400> 18
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Glu Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Asp Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 19
   <211> 807
   <212> DNA
   <213> Bacillus lentus DSM 5483
<400> 19
   gcgcaatcag tgccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cgcagggctt gacattgtcg caccaggggt aaacgtgcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc 807
<210> 20
   <211> 807
   <212> DNA
   <213> Bacillus lentus DSM 5483 clone M130
<400> 20
   gcgcaaacag tgccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc                                     807
<210> 21
   <211> 807
   <212> DNA
   <213> Bacillus lentus DSM 5483 clone M131
<400> 21
   gcgcaaacaa tcccatgggg aattagccgt gtgcaagccc cggctgccca taaccgtgga 60
   ttgacaggtt ctggtgtaaa agttgctgtc ctcgatacag gtatttccac tcatccagac 120
   ttaaatattc gtggtggcgc tagctttgta ccaggggaac catccactca agatgggaat 180
   gggcatggca cgcatgtggc cgggacgatt gctgctttaa acaattcgat tggcgttctt 240
   ggcgtagcgc ctagtgcgga actatacgct gttaaagttt taggagccga cggtagaggt 300
   gcaatcagct cgattgccca agggttggaa tgggcaggga acaatggcat gcacgttgct 360
   aatttgagtt taggaagccc ttcgccaagt gccacacttg agcaagctgt taatagcgcg 420
   acttctagag gcgttcttgt tgtagcggca tctgggaatt caggtgcaag ctcaatcagc 480
   tatccggccc gttatgcgaa cgcaatggca gtcggagcta ctgaccaaaa caacaaccgc 540
   gccagctttt cacagtatgg cccagggctt gacattatgg caccaggggt aaacattcag 600
   agcacatacc caggttcaac gtatgccagc ttaaacggta catcgatggc tactcctcat 660
   gttgcaggtg cagcagccct tgttaaacaa aagaacccat cttggtccaa tgtacaaatc 720
   cgcaaccatc taaagaatac ggcaacgagc ttaggaagca cgaacttgta tggaagcgga 780
   cttgtcaatg cagaagcggc aacacgc                                     807
<210> 22
   <211> 269
   <212> PRT
   <213> Bacillus lentus DSM 5483
<400> 22
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 23
   <211> 269
   <212> PRT
   <213> Bacillus lentus DSM 5483 clone M130
<400> 23
   Ala Gln Thr Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Pro Gly Leu Asp Ile
               180                 185                 190
   Met Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 24
   <211> 269
   <212> PRT
   <213> Bacillus lentus DSM 5483 clone M131
<400> 24
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Pro Gly Leu Asp Ile
   180 185 190
   Met Ala Pro Gly Val Asn Ile Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 25
   <211> 1727
   <212> DNA
   <213> Bacillus licheniformis ATCC 53926
<220>
   <221> 5'UTR
   <222> (240)..(313)
<220>
   <221> CDS
   <222> (314)..(1450)
<220>
   <221> sig_peptide
   <222> (314)..(397)
<220>
   <221> mat_peptide
   <222> (626)..(1447)
<220>
   <221> terminator
   <222> (1394)..(1563)
<220>
   <221> 3'UTR
   <222> (1451)..(1727)
<220>
   <221> misc_feature
   <222> (84)..(89)
   <223> restriction site Ava I
<220>
   <221> misc_feature
   <222> (376)..(381)
   <223> restriction site Cla I
<220>
   <221> misc_feature
   <222> (638)..(643)
   <223> restriction site Nco I
<220>
   <221> misc_feature
   <222> (950)..(955)
   <223> restriction site Kpn I
<220>
   <221> misc_feature
   <222> (1394)..(1399)
   <223> restriction site Kpn I
<220>
   <221> misc_feature
   <222> (1558)..(1563)
   <223> restriction site Kpn I
<400> 25
   gtctcctgga tctcataaaa taaatgaata gtattttcat aaaatgaatc atatggatgc 60
   aatctcctgt cattctgctg gccctcggga cctctttccc tcggaggctg aagcggtcta 120
   ttcatacttt cgaactgaac atttttctaa aacagttatt aataaccaaa aaattttaaa 180
   ttggtcctcc aaaaaaatag gcctaccata taattcattt tttttctata ataaattaac 240
   agaataattg gaatagatta tattatcctt ctatttaaat tattctgaat aaagaggagg 300
   agagtgagta atg atg agg aaa aag agt ttt tgg ctt ggg atg ctg acg    349
                 Met Arg Lys Lys Ser Phe Trp Leu Gly Met Leu Thr
                                     -100                -95
   gcc ttc atg ctc gtg ttc acg atg gca tcg atc gat tcc gct tct gct   397
   Ala Phe Met Leu Val Phe Thr Met Ala Ser Ile Asp Ser Ala Ser Ala
           -90                 -85                 -80
   gct caa ccg gcg aaa aat gtt gaa aag gat tat att gtc gga ttt aag    445
   Ala Gln Pro Ala Lys Asn Val Glu Lys Asp Tyr Ile Val Gly Phe Lys
       -75                 -70                 -65
   tca gga gtg aaa acc gca tct gtc aaa aag gac atc atc aaa gag agc    493
   Ser Gly Val Lys Thr Ala Ser Val Lys Lys Asp Ile Ile Lys Glu Ser
   -60                 -55                 -50                 -45
   ggc gga aaa gtg gac aag cag ttt aga atc atc aac gcg gca aaa gcg    541
   Gly Gly Lys Val Asp Lys Gln Phe Arg Ile Ile Asn Ala Ala Lys Ala
                   -40                 -35                 -30
   aag cta gac aaa gaa gcg ctt aag gaa gtc aaa aat gat ccg gat gtc    589
   Lys Leu Asp Lys Glu Ala Leu Lys Glu Val Lys Asn Asp Pro Asp Val
               -25                 -20                 -15
   gct tat gtg gaa gag gat cat gtg gcc cat gcc ttg gcg caa acc gtt    637
   Ala Tyr Val Glu Glu Asp His Val Ala His Ala Leu Ala Gln Thr Val
           -10                 -5              -1   1
   cca tgg ggc att cct ctc att aaa gcg gac aaa gtg cag gct caa ggc    685
   Pro Trp Gly Ile Pro Leu Ile Lys Ala Asp Lys Val Gln Ala Gln Gly
    5                  10                  15                  20
   ttt aag gga gcg aat gta aaa gta gcc gtc ctg gat aca gga atc caa    733
   Phe Lys Gly Ala Asn Val Lys Val Ala Val Leu Asp Thr Gly Ile Gln
                   25                  30                  35
   gct tct cat ccg gac ttg aac gta gtc ggc gga gca agc ttt gtg gct    781
   Ala Ser His Pro Asp Leu Asn Val Val Gly Gly Ala Ser Phe Val Ala
               40                  45                  50
   ggc gaa gct tat aac acc gac ggc aac gga cac ggc aca cat gtt gcc    829
   Gly Glu Ala Tyr Asn Thr Asp Gly Asn Gly His Gly Thr His Val Ala
           55                  60                  65
   ggt aca gta gct gcg ctt gac aat aca acg ggt gta tta ggc gtt gcg    877
   Gly Thr Val Ala Ala Leu Asp Asn Thr Thr Gly Val Leu Gly Val Ala
       70                  75                  80
   cca agc gta tcc ttg tac gcg gtt aaa gta ctg aat tca agc gga agc    925
   Pro Ser Val Ser Leu Tyr Ala Val Lys Val Leu Asn Ser Ser Gly Ser
    85                 90                  95                 100
   gga tca tac agc ggc att gta agc ggt acc gag tgg gcg aca aca aac    973
   Gly Ser Tyr Ser Gly Ile Val Ser Gly Thr Glu Trp Ala Thr Thr Asn
                   105                 110                 115
   ggc atg gat gtt atc aat atg agc ctt ggg gga gca tca ggc tcg aca    1021
   Gly Met Asp Val Ile Asn Met Ser Leu Gly Gly Ala Ser Gly Ser Thr
               120                 125                 130
   gcg atg aaa cag gca gtc gac aat gca tat gca aga ggg gtt gtc gtt    1069
   Ala Met Lys Gln Ala Val Asp Asn Ala Tyr Ala Arg Gly Val Val Val
           135                 140                 145
   gta gct gca gca ggg aac agc gga tct tca gga aac acg aat aca att    1117
   Val Ala Ala Ala Gly Asn Ser Gly Ser Ser Gly Asn Thr Asn Thr Ile
       150                 155                 160
   ggc tat cct gca aaa tac gat tct gtc atc gct gtt ggt gcg gta gac    1165
   Gly Tyr Pro Ala Lys Tyr Asp Ser Val Ile Ala Val Gly Ala Val Asp
   165                 170                 175                 180
   tct aac agc aac aga gct tca ttt tcc agc gtc gga gca gag ctt gaa    1213
   Ser Asn Ser Asn Arg Ala Ser Phe Ser Ser Val Gly Ala Glu Leu Glu
                   185                 190                 195
   gtc atg gct cct ggc gca ggc gta tac agc act tac cca acg aac act    1261
   Val Met Ala Pro Gly Ala Gly Val Tyr Ser Thr Tyr Pro Thr Asn Thr
               200                 205                 210
   tat gca aca ttg aac gga acg tca atg gct tct cct cat gta gcg gga    1309
   Tyr Ala Thr Leu Asn Gly Thr Ser Met Ala Ser Pro His Val Ala Gly
           215                 220                 225
   gca gca gct ttg atc ttg tca aaa cat ccg aac ctt tca gct tca caa    1357
   Ala Ala Ala Leu Ile Leu Ser Lys His Pro Asn Leu Ser Ala Ser Gln
       230                 235                 240
   gtc cgc aac cgt ctc tcc agc acg gcg act tat ttg ggt acc tcc ttc    1405
   Val Arg Asn Arg Leu Ser Ser Thr Ala Thr Tyr Leu Gly Thr Ser Phe
   245                 250                 255                 260
   tac tat ggg aaa ggt ctg atc aat gtc gaa gct gcc gct caa taa        1450
   Tyr Tyr Gly Lys Gly Leu Ile Asn Val Glu Ala Ala Ala Gln
                   265                 270                 275
   catattctaa caaatagcat atagaaaaag ctagtgtttt tagcactagc tttttcttca 1510
   ttctgatgaa ggttgttcaa tattttgaat ccgttccatg atcgtcgggt acccgtattt 1570
   aaaaatcttg acgagaaacg gcgggtttgc ctcgctcagc ccggcttttg agagctcttg 1630
   aaacgtcgaa accgctgcat cgctgttttg cgtcagttca atcgcatact ggtcagcagc 1690
   ttttcctgat gcctgaaact gctgctaaat gagacga 1727
<210> 26
   <211> 378
   <212> PRT
   <213> Bacillus licheniformis ATCC 53926
<400> 26

Further aspects generally relating to the technical field of the invention are:
1. A mutant subtilisin protease, characterized by at least one amino acid alteration which results in a reduced positive charge or an increased negative charge in the region of the substrate binding pocket, wherein said amino acid alteration is L211D according to the counting of SEQ ID NO. 22..
2. Mutant protease according to no. 1, derived from the protease described by SEQ ID NO. 22 by the following amino acid alteration: L211 D.
3. Mutant protease according to no. 1, derived from the protease described by SEQ ID NO. 24 by the following amino acid alteration: L211 D, preferably a protease described by SEQ ID NO. 16, SEQ ID NO. 17 or SEQ ID NO. 18.
4. Mutant protease according to no. 1, derived from the protease described by SEQ ID NO 23 by one of the following additional amino acid alterations: R99G, R99A or R99S.
5. Gene which codes for a mutant protease according to one of no. 1 to 4, preferably described by SEQ ID NO. 7, SEQ ID NO. 8 or SEQ ID NO. 9.
6. Hybrid gene, comprising, in the direction of transcription, a promotor, a ribosomal binding site, an initiation codon, the major portion of the pre region of SEQ ID NO. 25 oparably linked to a portion of a pre region and all of the pro and mature regions of the respective gene according to no. 5 followed by the transcription terminator sequence of the protease gene SEQ ID NO. 25.
7. Plasmid capable of replication in species of the genus Bacillus wherein said plasmid carries a mutant protease gene according to no. 6.
8. A transformed host of the genus Bacillus comprising a plasmid according to no. 7.
9. A transformed Bacillus host cell according to no. 8 wherein said host cell is *Bacillus licheniformis* ATCC 53926.
10. A transformed Bacillus host cell according to no. 8 wherein said host cell is *Bacillus subtilis.*
11. Detergent composition comprising at least one surfactant and a mutated subtilisin protease according to one of no. 1 to 4.
12. A detergent composition according to no. 11 comprising a proteolytically active amount of said mutated protease wherein said protease has a keracinase/caseinase activity ratio (KC ratio) of less than 0.80, preferably of less than 0.70, further preferably between 0.2 and 0.65.
13. A detergent composition according to no. 12 for washing fabrics composed of proteinogenic fibers.
14. A detergent composition according to one of no. 11 to 13 wherein said protease has a proteolytic activity of from 100 PU/g to 7500 PU/g.
15. A detergent composition according to one of no. 11 to 14 where said protease is encoded by SEQ ID NO. 16, SEQ ID NO. 17 or SEQ ID NO. 18, preferably SEQ ID NO. 16.
16. A detergent composition according to one of no. 11 to 15 wherein said at least one surfactant comprises a nonionic surfactant selected from the group consisting of a fatty alkyl polyglycoside, a fatty alkyl polyalkoxylate, a fatty acid polyhydroxamide, an ethoxylated fatty alkylamine, a propoxylated fatty alkylamine, an ethoxylated vicinal diol, a propoxylated vicinal diol, an ethoxylated fatty acid alkyl ester, a propoxylated fatty acid alkyl ester, an ethoxylated fatty acid amide, a propoxylated fatty acid amide, and mixtures thereof.
17. A detergent composition according to no. 16 wherein said nonionic surfactant is present in an amount from 2% to 25% by weight.
18. A detergent composition according to no. 16 or 17 further comprising an anionic surfactant selected from the group consisting of a fatty alkyl sulfate, a fatty alkyl ether sulfate, a sulfofatty acid ester, a sulfofatty acid disalt, and combinations thereof.
19. A detergent composition according to one of no. 11 to 15 wherein said at least one surfactant is comprising an anionic surfactant selected from the group consisting of a fatty alkyl sulfate, a fatty alkyl ether sulfate, a sulfofatty acid ester, a sulfofatty acid disalt, and combinations thereof.
20. A detergent composition according to no. 18 or 19 wherein said anionic surfactant is present in an amount from 2% to 25% by weight.
21. A detergent composition according to one of no. 18 to 20 wherein said anionic surfactant is selected from the group consisting of an alkyl sulfate, an alkenyl sulfate, an alkyl ether sulfate, an alkenyl ether sulfate wherein the alkyl or alkenyl group contains from 8 to 22 carbon atoms, preferably from 12 to 18 carbon atoms.
22. A detergent composition according to one of no. 11 to 21 further comprising a builder selected from the group consisting of an alkali metal alumosilicate, a crystalline alkali metal silicate with a modulus above 1, a monomeric polycarboxylate, a polymeric polycarboxylate and mixtures thereof.
23. A detergent composition according to no. 22 wherein said builder is present in an amount of from 2.5% to 60% by weight.
24. A detergent composition according to one of no. 11 to 23 further comprising from 20% to 55% by weight of an inorganic builder; up to 15% by weight of a water-soluble organic builder; from 2.5% to 20% by weight of an anionic surfactant; from 1% to 20% by weight of a nonionic surfactant; up to 25% by weight of a bleaching agent; up to 8% by weight of a bleach activator; up to 20% by weight of an inorganic salt; from 0.4% to 1.2% by weight of enzymes, preferably lipases, cutinases, amylases, cellulases, pullulanases, oxidases, peroxidases or mixtures thereof.
25. A detergent composition according to no. 24 wherein said inorganic salt is an alkali metal carbonate, an alkali metal hydrogen carbonate, or an alkali metal sulfate.
26. A detergent composition according to one of no. 11 to 25 additionally comprising a second mutant subtilisin protease, wherein said second mutant protease is characterized by at least one amino acid alteration which results in a reduced positive charge or an increased negative charge in the region of the substrate binding pocket, and is selected from the group of the amino acid alterations R99D, R99E, R99G, R99A, R99S according to the counting of SEQ ID NO. 22, or is a variant with the aminoacid alterations S3T/V4I/S154E/A188P/V193M/V199I or S3T/V4I/S1S4D/A188P/V193M/V199I.
27. A detergent composition according to no. 26 where said first mutant protease is encoded by the amino acid sequence of SEQ ID NO. 16.
28. A detergent composition according to one of no. 26 to 27 where said second mutant protease is encoded by the amino acid sequence of SEQ ID NO. 14.
29. A composition comprising a first and a second mutant subtilisin protease, wherein said first mutant protease is a mutant protease according to no. 1-4 and wherein said second mutant protease is characterized by at least one amino acid alteration which results in a reduced positive charge or an increased negative charge in the region of the substrate binding pocket, selected from the group of the amino acid alterations R99D, R99E, R99G, R99A, R99S according to the counting of SEQ ID NO. 22, or is a variant with the aminoacid alterations S3TN41/S154EIA188P/V193M/V1991 or S3T/V41/S154D/A188P/V193M/V199I.
30. A composition according to no. 29 where said first mutant protease is encoded by the amino acid sequence of SEQ ID NO. 16.
31. A composition according to no. 29 or 30 where said second mutant protease is encoded by the amino acid sequence of SEQ ID NO. 14.
32. A method for washing fabrics comprising contacting said fabric with a detergent composition according to one of no. 11 to 28.
33. A method for washing fabrics according to no. 32 wherein said fabrics are composed of proteinogenic fibers.
34. A method according to no. 32 or 33 for removing stains from cloth having both blood and egg stains.

## Claims

1. Detergent composition for removal of egg stains from fabrics, comprising at least one surfactant and a mutated subtilisin protease wherein said protease is encoded by SEQ ID NO. 16.

2. A detergent composition according to claim 1 comprising a proteolytically active amount of said mutated protease wherein said protease has a keratinase/caseinase activity ratio (KC ratio) of less than 0.80, preferably of less than 0.70.

3. A detergent composition according to claim 1 for washing fabrics composed of proteinogenic fibers.

4. A detergent composition according to one of claims 1 to 3 wherein said at least one surfactant comprises a nonionic surfactant selected from the group consisting of a fatty alkyl polyglycoside, a fatty alkyl polyalkoxylate, a fatty acid polyhydroxamide, an ethoxylated fatty alkylamine, a propoxylated fatty alkylamine, an ethoxylated vicinal diol, a propoxylated vicinal diol, an ethoxylated fatty acid alkyl ester, a propoxylated fatty acid alkyl ester, an ethoxylated fatty acid amide, a propoxylated fatty acid amide, and mixtures thereof.

5. A detergent composition according to claim 4 wherein said nonionic surfactant is present in an amount from 2% to 25% by weight.

6. A detergent composition according to claim 4 or 5 further comprising an anionic surfactant selected from the group consisting of a fatty alkyl sulfate, a fatty alkyl ether sulfate, a sulfofatty acid ester, a sulfofatty acid disalt, and combinations thereof.

7. A detergent composition according to one of claims 1 to 3 wherein said at least one surfactant is comprising an anionic surfactant selected from the group consisting of a fatty alkyl sulfate, a fatty alkyl ether sulfate, a sulfofatty acid ester, a sulfofatty acid disalt, and combinations thereof.

8. A detergent composition according to claim 6 or 7 wherein said anionic surfactant is present in an amount from 2% to 25% by weight.

9. A detergent composition according to one of claims 6 to 8 wherein said anionic surfactant is selected from the group consisting of an alkyl sulfate, an alkenyl sulfate, an alkyl ether sulfate, an alkenyl ether sulfate wherein the alkyl or alkenyl group contains from 8 to 22 carbon atoms, preferably from 12 to 18 carbon atoms.

10. A detergent composition according to one of claims 1 to 9 further comprising a builder selected from the group consisting of an alkali metal alumosilicate, a crystalline alkali metal silicate with a modulus above 1, a monomeric polycarboxylate, a polymeric polycarboxylate and mixtures thereof.

11. A detergent composition according to claim 10 wherein said builder is present in an amount of from 2.5% to 60% by weight.

12. A detergent composition according to one of claims 1 to 11 further comprising from 20% to 55% by weight of an inorganic builder; up to 15% by weight of a water-soluble organic builder; from 2.5% to 20% by weight of an anionic surfactant; from 1% to 20% by weight of a nonionic surfactant; up to 25% by weight of a bleaching agent; up to 8% by weight of a bleach activator; up to 20% by weight of an inorganic salt; from 0.4% to 1.2% by weight of enzymes, preferably lipases, cutinases, amylases, cellulases, pullulanases, oxidases, peroxidases or mixtures thereof.

13. A detergent composition according to claim 12 wherein said inorganic salt is an alkali metal carbonate, an alkali metal hydrogen carbonate, or an alkali metal sulfate.

14. A method for washing fabrics for removing egg stains comprising contacting said fabric with a detergent composition according to one of claims 1 to 13.

15. A method for washing fabrics according to claim 14 wherein said fabrics are composed of proteinogenic fibers.

## Patentansprüche

1. Waschmittel zur Entfernung von Eiflecken von textilen Flächengebilden, umfassend mindestens ein Tensid und eine mutierte Subtilisin-Protease, wobei die Protease durch SEQ ID NO. 16 codiert ist.

2. Waschmittel nach Anspruch 1, umfassend eine proteolytisch wirksame Menge der mutierten Protease, wobei die Protease ein Keratinase/Caseinase-Aktivitätsverhältnis (KC-Verhältnis) von weniger als 0,80, vorzugsweise weniger als 0,70, aufweist.

3. Waschmittel nach Anspruch 1 zum Waschen von textilen Flächengebilden aus proteinogenen Fasern.

4. Waschmittel nach einem der Ansprüche 1 bis 3, wobei das mindestens eine Tensid ein nichtionisches Tensid aus der Gruppe bestehend aus einem Fettalkylpolyglykosid, einem Fettalkylpolyalkoxylat, einem Fettsäurepolyhydroxamid, einem ethoxylierten Fettalkylamin, einem propoxylierten Fettalkylamin, einem ethoxylierten vicinalen Diol, einem propoxylierten vicinalen Diol, einem ethoxylierten Fettsäurealkylester, einem propoxylierten Fettsäurealkylester, einem ethoxylierten Fettsäureamid, einem propoxylierten Fettsäureamid und Mischungen davon umfasst.

5. Waschmittel nach Anspruch 4, wobei das nichtionische Tensid in einer Menge von 2 bis 25 Gew.-% vorliegt.

6. Waschmittel nach Anspruch 4 oder 5, ferner umfassend ein anionisches Tensid aus der Gruppe bestehend aus einem Fettalkylsulfat, einem Fettalkylethersulfat, einem Sulfofettsäureester, einem Sulfofettsäuredisalz und Kombinationen davon.

7. Waschmittel nach einem der Ansprüche 1 bis 3, wobei das mindestens eine Tensid ein anionisches Tensid aus der Gruppe bestehend aus einem Fettalkylsulfat, einem Fettalkylethersulfat, einem Sulfofettsäureester, einem Sulfofettsäuredisalz und Kombinationen davon umfasst.

8. Waschmittel nach Anspruch 6 oder 7, wobei das anionische Tensid in einer Menge von 2 bis 25 Gew.-% vorliegt.

9. Waschmittel nach einem der Ansprüche 6 bis 8, wobei das anionische Tensid aus der Gruppe bestehend aus einem Alkylsulfat, einem Alkenylsulfat, einem Alkylethersulfat und einem Alkenylethersulfat ausgewählt ist, wobei die Alkyl- bzw. Alkenylgruppe 8 bis 22 Kohlenstoffatome, vorzugsweise 12 bis 18 Kohlenstoffatome, enthält.

10. Waschmittel nach einem der Ansprüche 1 bis 9, ferner umfassend einen Builder aus der Gruppe bestehend aus einem Alkalimetallalumosilicat, einem kristallinen Alkalimetallsilicat mit einem Modul über 1, einem monomeren Polycarboxylat, einem polymeren Polycarboxylat und Mischungen davon.

11. Waschmittel nach Anspruch 10, wobei der Builder in einer Menge von 2,5 bis 60 Gew.-% vorliegt.

12. Waschmittel nach einem der Ansprüche 1 bis 11, ferner umfassend 20 bis 55 Gew.-% eines anorganischen Builders; bis zu 15 Gew.-% eines wasserlöslichen organischen Builders; 2,5 bis 20 Gew.-% eines anionischen Tensids; 1 bis 20 Gew.-% eines nichtionischen Tensids; bis zu 25 Gew.-% eines Bleichmittels; bis zu 8 Gew.-% eines Bleichaktivators; bis zu 20 Gew.-% eines anorganischen Salzes; 0,4 bis 1,2 Gew.-% Enzyme, vorzugsweise Lipasen, Cutinasen, Amylasen, Cellulasen, Pullulanasen, Oxidasen, Peroxidasen oder Mischungen davon.

13. Waschmittel nach Anspruch 12, wobei es sich bei dem anorganischen Salz um ein Alkalimetallcarbonat, ein Alkalimetallhydrogencarbonat oder ein Alkalimetallsulfat handelt.

14. Verfahren zum Waschen von textilen Flächengebilden zur Entfernung von Eiflecken, bei dem man das textile Flächengebilde mit einem Waschmittel nach einem der Ansprüche 1 bis 13 in Berührung bringt.

15. Verfahren zum Waschen von textilen Flächengebilden nach Anspruch 14, wobei die textilen Flächengebilde aus proteinogenen Fasern bestehen.

## Revendications

1. Composition détergente destinée à éliminer des taches d'oeuf de tissus, comprenant au moins un tensioactif et une protéase de subtilisine ayant subi une mutation, ladite protéase étant codée par la SÉQ ID n° 16.

2. Composition détergente selon la revendication 1, comprenant une quantité protéolytiquement active de ladite protéase ayant subi une mutation, ladite protéase ayant un rapport d'activité de kératinase/caséinase (rapport KC) inférieur à 0,80, préférablement inférieur à 0,70.

3. Composition détergente selon la revendication 1, destinée au lavage de tissus constitués de fibres protéinogènes.

4. Composition détergente selon l'une des revendications 1 à 3, dans laquelle ledit au moins un tensioactif comprend un tensioactif non ionique sélectionné dans le groupe constitué d'un alkylpolyglycoside gras, d'un alkylpolyalcoxylate gras, d'un polyhydroxamide d'acide gras, d'une alkylamine grasse éthoxylée, d'une alkylamine grasse propoxylée, d'un diol vicinal éthoxylé, d'un diol vicinal propoxylé, d'un ester alkylique d'acide gras éthoxylé, d'un ester alkylique d'acide gras propoxylé, d'un amide d'acide gras éthoxylé, d'un amide d'acide gras propoxylé, et de mélanges de ceux-ci.

5. Composition détergente selon la revendication 4, dans laquelle ledit tensioactif non ionique est présent dans une quantité de 2 % à 25 % en poids.

6. Composition détergente selon la revendication 4 ou 5, comprenant en outre un tensioactif anionique sélectionné dans le groupe constitué d'un alkylsulfate gras, d'un alkyléthersulfate gras, d'un ester d'acide gras sulfo, d'un double sel d'acide gras sulfo, et de combinaisons de ceux-ci.

7. Composition détergente selon l'une des revendications 1 à 3, dans laquelle ledit au moins un tensioactif comprend un tensioactif anionique sélectionné dans le groupe constitué d'un alkylsulfate gras, d'un alkyléthersulfate gras, d'un ester d'acide gras sulfo, d'un double sel d'acide gras sulfo, et de combinaisons de ceux-ci.

8. Composition détergente selon la revendication 6 ou 7, dans laquelle ledit tensioactif anionique est présent dans une quantité de 2 % à 25 % en poids.

9. Composition détergente selon l'une des revendications 6 à 8, dans laquelle ledit tensioactif anionique est sélectionné dans le groupe constitué d'un alkylsulfate, d'un alcénylsulfate, d'un alkyléthersulfate, d'un alcényléthersulfate où le groupe alkyle ou alcényle comprend 8 à 22 atomes de carbone, préférablement 12 à 18 atomes de carbone.

10. Composition détergente selon l'une des revendications 1 à 9, comprenant en outre un builder sélectionné dans le groupe constitué d'un aluminosilicate de métal alcalin, d'un silicate de métal alcalin cristallin avec un module supérieur à 1, d'un polycarboxylate monomère, d'un polycarboxylate polymère, et de mélanges de ceux-ci.

11. Composition détergente selon la revendication 10, dans laquelle ledit builder est présent dans une quantité de 2,5 % à 60 % en poids.

12. Composition détergente selon l'une des revendications 1 à 11, comprenant en outre de 20 % en poids à 55 % en poids d'un builder inorganique ; jusqu'à 15 % en poids d'un builder organique hydrosoluble ; de 2,5 % en poids à 20 % en poids d'un tensioactif anionique ; de 1 % en poids à 20 % en poids d' un tensioactif non ionique ; jusqu'à 25 % en poids d' un agent de blanchiment ; jusqu'à 8 % en poids d'un activateur de blanchiment ; jusqu'à 20 % en poids d'un sel inorganique ; de 0,4 % en poids à 1,2 % en poids d'enzymes, de préférence des lipases, des cutinases, des amylases, des cellulases, des pullulanases, des oxydases, des peroxydases ou des mélanges de celles-ci.

13. Composition détergente selon la revendication 12, dans laquelle ledit sel inorganique est un carbonate de métal alcalin, un hydrogénocarbonate de métal alcalin ou un sulfate de métal alcalin.

14. Procédé pour le lavage de tissus afin d'éliminer les taches d'oeuf, comprenant la mise en contact dudit tissu avec une composition détergente selon l'une quelconque des revendications 1 à 13.

15. Procédé pour le lavage de tissus selon la revendication 14, dans lequel lesdits tissus sont constitués de fibres protéinogènes.
